# EUROPEAN PATENT APPLICATION

(11) **EP 4 570 906 A1**
(43) Date of publication of application: **18.06.2025**
(21) Application number: 23851902.9
(22) Date of filing: 10.08.2023
(51) Int. Cl.: C12N 9/22, C12N 15/864, C07K 14/47, A61K 9/00

(54) **METHOD FOR EPITOPE EDITING TARGET AND USE**

(30) Priority: 11.08.2022 WO PCT/CN2022/111647
(71) Applicant: Epigenic Therapeutics Inc., Shanghai 200131 (CN)
(72) Inventor: ZHANG, Baohong, Shanghai 200131 (CN); LUO, Hao, Shanghai 200131 (CN); LV, Ruimin, Shanghai 200131 (CN); YANG, Changqing, Shanghai 200131 (CN)
(74) Representative: Engelhard, Markus
(86) International application number: PCT/CN2023/112097
(87) International publication number: WO 2024/032681

(57) **Abstract**

Provided are a method for adjusting the expression level of a target gene and a nucleic acid binding molecule. The combination of the nucleic acid binding molecule and a gene expression regulation molecule has the ability of adjusting the expression level of the target gene.

## Description

### TECHNICAL FIELD

The present application relates to the field of biomedicine, and specifically relates to an epigenetic editing target and use thereof.

### BACKGROUND

Fusion of catalytically inactive, "dead Cas9 (dCas9)" to the Kruppel-associated box (KRAB) domain generates a gene expression repressor capable of highly specific and effective regulating or silencing of target genes in cell culture experiments. However, in *in vivo* environment, the repressor faces certain challenges in exerting therapeutic effects. For example, safety, toxicity, immunogenicity, and off-target effects are other challenges that limit the *in vivo* use of synthetic repressors.

Therefore, there is a need in this art for an effective epigenetic editing target, wherein targeting said target can be used to improve the epigenetic editing effect and safety, and to reduce toxicity, immunogenicity, and/or off-target effects.

### SUMMARY

The present application provides an epigenetic editing target, wherein targeting said target can be used to improve the epigenetic editing effect and safety, and to reduce toxicity, immunogenicity, and/or off-target effects. For example, by targeting the vicinity of the target gene and/or the within of a regulatory element of the target gene, at least one nucleotide modification can be effectively performed so as to regulate (for example, reduce or eliminate) the expression of the product of the target gene in cells.

In an aspect, the present application provides a method for regulating the expression and/or activity of the LPA (Lipoprotein(A)) gene, comprising providing a gene expression-regulating molecule or a nucleic acid encoding the gene expression-regulating molecule, wherein the gene expression-regulating molecule has the function of regulating the expression of the LPA gene without altering its gene sequence.

In another aspect, the present application provides a method for treating and/or alleviating a condition related to abnormal expression and/or activity of the LPA gene, comprising providing a gene expression-regulating molecule or a nucleic acid encoding the gene expression-regulating molecule, wherein the gene expression-regulating molecule has the function of regulating the expression of the LPA gene without altering its gene sequence.

In some embodiments, the gene expression-regulating molecule comprises a first functional domain providing modification of at least one nucleotide in the vicinity of the LPA gene and/or within the LPA gene regulatory elements.

In some embodiments, the modification of at least one nucleotide comprises methylation modification.

In some embodiments, the regulatory elements comprise a core promoter, a proximal promoter, a distal enhancer, a silencer, an insulator element, a boundary element, and/or a locus control region.

In some embodiments, the first functional domain comprises one or more of DNA methyltransferase, DNA demethylase, and a functional active fragment thereof.

In some embodiments, the DNA methyltransferase comprises one or more of DNMT 3A, DNMT 3B, DNMT 3L, DNMT 1, and DNMT 2.

In some embodiments, the DNMT 3A is derived from mouse.

In some embodiments, the DNMT 3L is derived from human and/or mouse.

In some embodiments, the DNMT 3A and the DNMT 3L are directly and/or indirectly linked.

In some embodiments, the gene expression-regulating molecule comprises a second functional domain comprising a zinc finger protein-based transcription factor or a functional active fragment thereof, or a substance capable of modifying histone.

In some embodiments, the second functional domain comprises Krab.

In some embodiments, the second functional domain comprises ZIM3 Krab or KOX1 Krab.

In some embodiments, the second functional domain comprises one or more of histone methyltransferase, histone demethylase, histone acetyltransferase, histone deacetylase, and a functional active fragment thereof.

In some embodiments, the gene expression-regulating molecule comprises the first functional domain and the second functional domain, with the first functional domain is directly or indirectly linked to one end of the second functional domain, or the first functional domain is directly and/or indirectly linked to both ends of the second functional domain.

In some embodiments, the gene expression-regulating molecule comprises a DNA binding domain.

In some embodiments, the gene expression-regulating molecule comprises one or more DNA binding domains selected from TALEN domain, zinc finger domain, and protein domain of a CRISPR/Cas system.

In some embodiments, the gene expression-regulating molecule comprises a Cas enzyme.

In some embodiments, the gene expression-regulating molecule comprises a Cas enzyme that substantially incapable of nuclease activity.

In some embodiments, the gene expression-regulating molecule comprises a dCas9 enzyme.

In some embodiments, the first functional domain and the second functional domain are directly or indirectly linked to one end of the DNA binding domain, or the first functional domain and the second functional domain are directly and/or indirectly linked to both ends of the DNA binding domain.

In some embodiments, the gene expression-regulating molecule is capable of binding to a DNA region within 500 bp upstream and/or downstream of the transcription start site (TSS) of the LPA gene, or a fragment thereof.

In some embodiments, the gene expression-regulating molecule is capable of binding to a DNA region where SEQ ID NO: 51 is located, or a fragment thereof.

In some embodiments, the gene expression-regulating molecule is capable of binding to one or more DNA regions in the vicinity of the transcription start site (TSS) of the LPA gene as described below: between 410 bp upstream and 380 bp upstream of the TSS, between 230 bp upstream and 140 bp upstream of the TSS, and between 260 bp downstream and 370 bp downstream of the TSS.

In some embodiments, the gene expression-regulating molecule is capable of binding to one or more DNA regions in the vicinity of the transcription start site (TSS) of the LPA gene as described below: between 410 bp upstream and 380 bp upstream of the TSS, between 230 bp upstream and 190 bp upstream of the TSS, between 170 bp upstream and 140 bp upstream of the TSS, between 260 bp downstream and 290 bp downstream of the TSS, and between 330 bp downstream and 370 bp downstream of the TSS.

In some embodiments, the method comprises providing a nucleic acid-binding molecule comprising a sequence of any one of SEQ ID NOs: 1-50.

In some embodiments, the gene expression-regulating molecule and/or the nucleic acid-binding molecule are formulated in a same delivery carrier or in different delivery carriers.

In some embodiments, the delivery carrier comprises liposome and/or lipid nanoparticle.

In some embodiments, the gene expression-regulating molecule and/or the nucleic acid-binding molecule are formulated in a same recombinant vector or in different recombinant vectors.

In some embodiments, the recombinant vector comprises viral vector.

In some embodiments, the recombinant vector comprises adeno-associated viral (AAV) vector.

In some embodiments, the gene expression-regulating molecule comprises a nuclear localization sequence.

In some embodiments, the nuclear localization sequence comprises amino acid with positively charged group.

In some embodiments, the nuclear localization sequence is located at the N-terminal and/or C-terminal of the first functional domain, the N-terminal and/or C-terminal of the second functional domain, and/or the N-terminal and/or C-terminal of the DNA binding domain.

In another aspect, the present application provides a nucleic acid-binding molecule, comprising a sequence of any one of SEQ ID NOs: 1-50.

In another aspect, the present application provides a gene expression-regulating molecule, having the function of regulating the expression of the LPA gene without altering its gene sequence.

In some embodiments, the gene expression-regulating molecule is the gene expression-regulating molecule provided in the method of the present application.

In some embodiments, the gene expression-regulating molecule and/or the nucleic acid-binding molecule are formulated in a same delivery carrier or in different delivery carriers, the nucleic acid-binding molecule comprises a sequence of any one of SEQ ID NOs: 1-50.

In some embodiments, the delivery carrier comprises liposome and/or lipid nanoparticle.

In some embodiments, the expression-regulating molecule and/or the nucleic acid-binding molecule are formulated in a same recombinant vector or in different recombinant vectors, the nucleic acid-binding molecule comprises a sequence of any one of SEQ ID NOs: 1-50.

In some embodiments, the recombinant vector comprises viral vector.

In some embodiments, the recombinant vector comprises adeno-associated viral (AAV) vector.

In another aspect, the present application provides a nucleic acid encoding the nucleic acid-binding molecule of the present application and/or encoding the gene expression-regulating molecule of the present application.

In another aspect, the present application provides a recombinant vector comprising the nucleic acid of the present application.

In another aspect, the present application provides a delivery carrier comprising the nucleic acid-binding molecule of the present application, the gene expression-regulating molecule of the present application, the nucleic acid of the present application, and/or the recombinant vector of the present application, and optionally comprising liposomes and/or lipid nanoparticles.

In another aspect, the present application provides a composition comprising the nucleic acid-binding molecule of the present application, the gene expression-regulating molecule of the present application, the nucleic acid of the present application, the recombinant vector of the present application, and/or the delivery carrier of the present application.

In another aspect, the present application provides a cell comprising the nucleic acid-binding molecule of the present application, the gene expression-regulating molecule of the present application, the nucleic acid of the present application, the recombinant vector of the present application, the delivery carrier of the present application, and/or the composition of the present application.

In another aspect, the present application provides a kit comprising the nucleic acid-binding molecule of the present application, the gene expression-regulating molecule of the present application, the nucleic acid of the present application, the recombinant vector of the present application, the delivery carrier of the present application, the composition of the present application, and/or the cell of the present application.

In another aspect, the present application provides a method for regulating the expression and/or activity of a target gene, comprising providing the nucleic acid-binding molecule of the present application, the gene expression-regulating molecule of the present application, the nucleic acid of the present application, the recombinant vector of the present application, the delivery carrier of the present application, the composition of the present application, the cell of the present application, and/or the kit of the present application.

In another aspect, the present application provides use of the nucleic acid-binding molecule of the present application, the gene expression-regulating molecule of the present application, the nucleic acid of the present application, the recombinant vector of the present application, the delivery carrier of the present application, the composition of the present application, the cell of the present application, and/or the kit of the present application in the preparation of a medicament for treating and/or alleviating a condition, said condition comprises a condition related to abnormal expression and/or activity of the target gene.

Those skilled in the art will be able to readily gain insight into other aspects and advantages of the present application from the detailed description below. The detailed description below only shows and describes exemplary embodiments of the present application. As those skilled in the art will recognize, the content of the present application enables those skilled in the art to make modifications to the specific embodiments disclosed without departing from the spirit and scope of the invention disclosed in the present application. Accordingly, the descriptions in the drawings and the specification are merely exemplary and are not intended to be limiting.

### BRIEF DESCRIPTION OF THE DRAWINGS

The specific features of the invention involved in the present application are shown in the appended claims. The features and advantages of the invention involved in the present application can be better understood by referring to the detailed description of the exemplary embodiments and the drawings provided below. A brief description of the drawings is as follows:

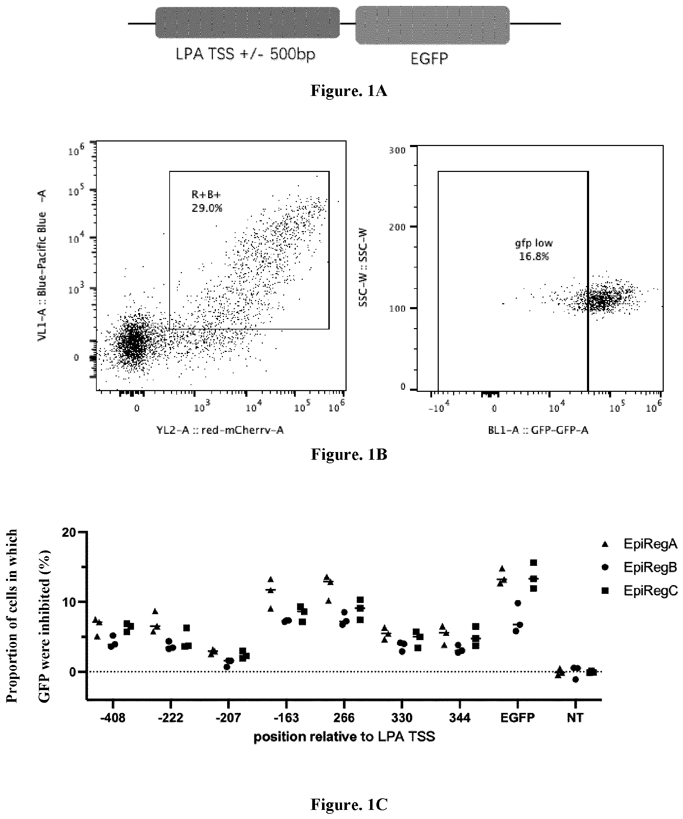

Figure 1A shows a schematic diagram of a reporter plasmid constructed by an LPA gene fragment and a fluorescent protein.
Figure 1B shows the results of flow cytometry detection of the proportion of cells with low green fluorescence intensity in a population of transfected cells.
Figure 1C shows the expression regulation effect of the gene expression-regulating molecules of the present application when targeting different regulatory regions of the LPA gene.

### DETAILED DESCRIPTION

The following embodiments of the present application invention are illustrated by particular specific examples, and other advantages and effects of the present application invention may be readily appreciated by those familiar with the art from what is disclosed in this specification.

### Definitions of Terms

In the present application, the terms "nucleic acid", "polynucleotide", "nucleotide", "nucleotide sequence", and "oligonucleotide" are used interchangeably and generally refer to nucleotides (e.g., deoxyribonucleotides or ribonucleotides), and polymers or complements thereof in single-stranded, double-stranded, or multi-stranded forms. For example, nucleotides can be ribonucleotides, deoxyribonucleotides, or modified versions thereof. For example, nucleotides can be single-stranded and double-stranded DNA, single-stranded and double-stranded RNA, and hybrid molecules of mixtures with single-stranded and double-stranded DNA and RNA. For example, nucleotides can include, but are not limited to, any type of RNA, such as mRNA, siRNA, miRNA, sgRNA, and guide RNA, as well as any type of DNA, genomic DNA, plasmid DNA, and minicircle DNA, and any fragments thereof. The term also covers nucleic acids containing known nucleotide analogs or modified backbone residues or bonds, which are synthetic, naturally occurring, and non-naturally occurring.

In the present application, the terms "sequence encoding..." or "nucleic acid encoding..." generally refer to nucleic acids (RNA or DNA molecules) that comprise nucleotide sequences encoding proteins. The encoding sequence may also include start and stop signals operably linked to regulatory elements, which comprise promoters and polyadenylation signals that can direct expression in the cells of individuals or mammals to which the nucleic acid is administered. The encoding sequence can be codon optimized.

In the present application, the term "treatment", for example, when used in the context of a disease, means that a subject (e.g., a human) who has a disease, is at risk of having a disease, and/or experiences a symptom of a disease, will, in an embodiment, suffer a less severver symptom and/or will recover faster, when administered a gene expression-regulating molecule or a nucleic acid encoding the gene expression-regulating molecule described herein and/or a nucleic acid-binding molecule (e.g., gRNA) or a nucleic acid encoding the nucleic acid-binding molecule described herein, compared to when such gene expression-regulating molecule or nucleic acid and/or nucleic acid-binding molecule or nucleic acid encoding such nucleic acid-binding molecule was never administered.

In the present application, the term "DNA binding domain" generally refers to independently folded protein domains that contain at least one motif recognizing double-stranded or single-stranded DNA. For example, the DNA binding domain may recognize a specific DNA sequence (recognition or regulatory sequence) or have general affinity for DNA. In some cases, other domains of the DNA binding domain typically regulate the activity of the DNA binding domain; DNA binding function can be structural or include transcription regulation, sometimes these two functions overlap. In some embodiments of the methods and gene expression-regulating molecules provided in the present application, the DNA binding domain may include (DNA) nucleases, such as nucleases that can target DNA in a sequence-specific manner or can be guided or directed to target DNA in a sequence-specific manner, such as CRISPR-Cas systems, zinc finger nucleases (ZFN), transcription activator-like effector nucleases (TALEN), or meganucleases. In some embodiments, the DNA binding domain is a DNA nuclease derived from the CRISPR-Cas system. For example, the DNA nuclease derived from the CRISPR-Cas system is a Cas protein.

In the present application, "Cas enzyme" can be used interchangeably with "Cas protein", "CRISPR protein", "CRISPR enzyme", "CRISPR-Cas protein", "CRISPR-Cas enzyme", "Cas", "CRISPR effector", or "Cas effector protein", which generally refers to a class of enzymes that are complementary to CRISPR sequences and can use CRISPR sequences as a guide to recognize and cleave specific DNA strands. Non-limiting examples of Cas proteins include Casl, CaslB, Cas2, Cas3, Cas4, Cas5, Cas6, Cas7, Cas8, Cas9(also known as Csnl and Csxl2), CaslO, Csyl, Csy2, Csy3, Csel, Cse2, Cscl, Csc2, Csa5, Csn2, Csm2, Csm3, Csm4, Csm5, Csm6, Cmrl, Cmr3, Cmr4, Cmr5, Cmr6, Csbl, Csb2, Csb3, Csxl7, Csxl4, CsxlO, Csxl6, CsaX, Csx3, Csxl, Csxl5, Csf1, Csf2, Csf3, Csf4, and/or their homologs, or modified forms. These proteins are known, for example, the amino acid sequence of the *Streptococcus pyogenes* Cas9 protein is availabe at the SwissProt database under the accession number Q99ZW2.

In the present application, the term "dCas9 enzyme" is also known as "dead Cas9 protein" or "dead Cas9 enzyme". Methods for generating Cas9 proteins (or fragments thereof) with inactivated DNA cleavage domains are known, for example, Jinek et al., Science. 337: 816-821(2012); Qi et al., "Repurposing CRISPR as an RNA-Guided Platform for Sequence-Specific Control of Gene Expression", Cell. 28, 152(5): 1173-83 (2013), the entire contents of which are incorporated herein by reference. For example, the DNA cleavage domain of Cas9 is known to includes two subdomains, HNH nuclease subdomain and RuvC1 subdomain. The HNH subdomain cleaves the strand complementary to the gRNA, while the RuvC1 subdomain cleaves the non-complementary strand. Mutations in these subdomains can silence the nuclease activity of Cas9. For example, mutations D10A and H840A completely inactivate the nuclease activity of *Streptococcus pyogenes* Cas9 (Jinek et al., Science. 337: 816-821(2012); Qi et al., Cell. 28; 152(5): 1173-83(2013)). Suitable CRISPR inactivated or nicked DNA binding domains include, but are not limited to, nuclease-inactivated mutations of Cas9 domains, including D10A, D10A/D839A/H840A, and D10A/D839A/H840A/N863A mutated domains, as described in WO2015089406A1, which is incorporated herein by reference. In some cases, dCas9 without endonuclease activity derived from *Streptococcus pyogenes* has been targeted to genes in bacteria, yeast, and human cells with gRNA to silence gene expression by steric hindrance. As used herein, "dCas" can refer to dCas protein or a fragment thereof. As used herein, "dCas9" can refer to dCas9 protein or a fragment thereof. As used herein, the terms "iCas" and "dCas" can be used interchangeably to refer to catalytically inactive CRISPR-associated proteins. In one embodiment, the dCas protein comprises one or more mutations in a DNA cleavage domain. In one embodiment, the dCas protein comprises one or more mutations in the RuvC or domain. In one embodiment, the dCas molecule includes one or more mutations in both the RuvC and HNH domains. In one embodiment, the dCas protein is a fragment of a wild-type Cas protein. In one embodiment, the dCas protein comprises a functional domain derived from a wild-type Cas protein, wherein the functional domain is selected from a Reel domain, a bridge helix domain, or a PAM interaction domain. In one embodiment, the nuclease activity of dCas is reduced by at least 40%, at least 45%, at least 50%, at least 55%, at least 60%, at least 65%, at least 70%, at least 75%, at least 80%, at least 85%, at least 90%, at least 95%, or at least 99% compared to the nuclease activity of a corresponding wild-type Cas protein.

Suitable dCas can be derived from wild-type Cas proteins. The Cas protein can be derived from Type I, II, or III CRISPR-Cas systems. In one embodiment, suitable dCas can be derived from Cas1, Cas2, Cas3, Cas4, Cas5, Cas6, Cas7, Cas8, Cas9, or Cas10. In one embodiment, dCas is derived from a Cas9 protein. For example, dCas9 can be obtained by introducing point mutations (e.g., substitutions, deletions, or additions) in the DNA cleavage domain (e.g., nuclease domain, e.g., RuvC and /or HNH domain) of the Cas9 protein. See, e.g., Jinek et al., Science (2012) 337: 816-21, which is incorporated herein by reference in its entirety. For example, introducing two point mutations in the RuvC and HNH domains reduces the Cas9 nuclease activity while retaining the Cas9 sgRNA and DNA binding activity. In one embodiment, the two point mutations within the RuvC and HNH active sites are the D10A and H840A mutations of *Streptococcus pyogenes* Cas9. Alternatively, D10 and H840 of *Streptococcus pyogenes* Cas9 can be deleted to abolish the Cas9 nuclease activity while retaining its sgRNA and DNA binding activity. In one embodiment, the two point mutations within the RuvC and HNH active sites are the D10A and N580A mutations of *Streptococcus pyogenes* Cas9.

In various embodiments, the present application involves a dCas protein, or any variant or mutant thereof. All variants and mutants of dCas9 can be used in a method, composition, fusion molecule, or kit disclosed herein, including but not limited to those derived from SpCas9 (Cas9 isolated from *Streptococcus pyogenes),* SaCas9 (Cas9 isolated from *Staphylococcus aureus),* StCas9 (Cas9 isolated from *Streptococcus thermophilus),* NmCas9 (Cas9 isolated from *Neisseria meningitidis),* FnCas9 (Cas9 isolated from *Francisella novicida*)*,* CjCas9 (Cas9 isolated from *Campylobacter jejuni),* ScCas9 (Cas9 isolated from *Streptococcus canis),* and any variants and mutants of the Cas9 listed above, such as high-fidelity Cas9 (Kleinstiver et al., Nature, January 28, 2016) and enhanced SpCas9 (Slaymaker et al., Sciences, January 1, 2016). For example, the dCas9 sequences shown in SEQ ID NOs: 1162-1179 in the present application are just a few exemplary options and are not exclusive. In one embodiment, the dCas protein is a *Streptococcus pyogenes* dCas9 protein with mutations at D10 and/or H840 (as shown in SEQ ID NO: 1162). In one embodiment, the dCas protein is *a Streptococcus pyogenes* dCas9 protein with D10A and/or H840A mutations (as shown in SEQ ID NO: 1162). In one embodiment, the dCas9 protein is a *Staphylococcus aureus* dCas9 protein, which comprises the amino acid sequence shown in SEQ ID NO: 1163 or 1164, a sequence that is substantially the same as SEQ ID NO: 1163 or 1164 (e.g., at least 80%, at least 85%, at least 90%, at least 91%, at least 92%, at least 93%, at least 94%, at least 95%, at least 96%, at least 97%, at least 98%, at least 99% or higher sequence identity), or a sequence with 1, 2, 3, 4, 5, or more modifications (e.g., amino acid substitutions, insertions, or deletions) relative to SEQ ID NO: 1163 or 1164, or any fragment thereof.

Similar mutations can also be applied to any other naturally occurring Cas9 (e.g., Cas9 from other species) or engineered Cas9. In some embodiments, dCas9 includes *Streptococcus pyogenes* dCas9, *Staphylococcus aureus* dCas9, *Campylobacter jejuni* dCas9, *Corynebacterium diphtheria* dCas9, *Eubacterium ventriosum* dCas9, *Streptococcus pasteurianus* dCas9, *Lactobacillus farciminis* dCas9, *Sphaerochaeta globus* dCas9, *Azospirillum* (e.g., strain B510) dCas9, *Gluconacetobacter diazotrophicus* dCas9, *Neisseria cinerea* dCas9, *Roseburia intestinalis* dCas9, *Parvibaculum lavamentivorans* dCas9, *Nitratifractor salsuginis* (e.g., strain DSM 16511) dCas9, *Campylobacter lari* (e.g., strain CF89-12) dCas9, *Streptococcus thermophilus* (e.g., strain LMD-9) dCas9, or fragments described above. In some embodiments, the present application also provides vectors encoding the following protein molecules: *Streptococcus pyogenes* dCas9, *Staphylococcus aureus* dCas9, *Campylobacter jejuni* dCas9, *Corynebacterium diphtheria* dCas9, *Eubacterium ventriosum* dCas9, *Streptococcus pasteurianus* dCas9, *Lactobacillus farciminis* dCas9, *Sphaerochaeta globus* dCas9, *Azospirillum* (strain B510) dCas9, *Gluconacetobacter diazotrophicus* dCas9, *Neisseria cinerea* dCas9, *Roseburia intestinalis* dCas9, *Parvibaculum lavamentivorans* dCas9, *Nitratifractor salsuginis* (strain DSM 16511) dCas9, *Campylobacter lari* (strain CF89-12) dCas9, *Streptococcus thermophilus* (strain LMD-9) dCas9, or fragments described above.

In the present application, the term "Cas enzyme with substantially no nuclease activity" generally refers to an RNA-guided enzyme in which recognition of phosphodiester bonds is facilitated by a separate polynucleotide sequence (e.g., guide RNA), but the enzyme may not significantly cleave the target phosphodiester bonds (e.g., no measurable phosphodiester bond cleavage under physiological conditions). For example, when complexed with a polynucleotide (e.g., sgRNA), an RNA-guided DNA endonuclease lacking nuclease activity retains DNA binding capability (e.g., specific binding to the target sequence) but lacks significant endonuclease activity. For example, an RNA-guided DNA endonuclease lacking nuclease activity is dCas9, ddCpf1, Cas9 variants lacking nuclease activity, or Class II CRISPR endonucleases lacking nuclease activity. For example, an RNA-guided DNA endonuclease lacking nuclease activity is dCas9. As mentioned herein, the term "dCas9" or "dCas9 protein" refers to a Cas9 protein with defects or lack of activity in both catalytic sites of the endonuclease activity. For example, dCas9 substantially lacks detectable endonuclease (e.g., deoxyriboendonuclease) activity. In all aspects, for example, dCas9 includes variants or homologs with an amino acid sequence that has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity with the dCas9 enzyme sequence of the present application.

In the present application, the term "capable of binding" can be used interchangeably with "binding to", "specifically recognizing", "targeting", etc., and generally refers to the binding molecule (e.g., the gene expression-regulating molecule of the present application) being able to interact with nucleotides on the target gene or target site, or the binding molecule (e.g., the gene expression-regulating molecule of the present application) having sufficient affinity for the target gene or target site, and this interaction can be through conjugation, coupling, attachment, providing complementarity, providing covalent forces, providing non-covalent forces, enhancing binding stability, etc.

In the present application, the term "transcription start site" generally refers to the nucleic acid in a construct, which corresponds to a first nucleic acid integrated into the primary transcript (i.e., mRNA precursor); the transcription start site can overlap with the promoter sequence.

In the present application, the term "fragment thereof" generally refers to a part or fragment of a specified whole. For example, when used in the present application with respect to a specified nucleotide sequence, the term "fragment thereof" refers to a continuous length of a specified nucleotide sequence that is shorter than the full-length sequence of the specified polynucleotide. A part of a specified nucleotide can be defined by its first position and its last position, wherein the first and last positions correspond to positions in the sequence of the specified polynucleotide, with the sequence position corresponding to the first position being at the N-terminal side of the sequence position corresponding to the last position, and thus the sequence of the part is a continuous nucleotide sequence in the specified polynucleotide, starting at the sequence position corresponding to the first position and ending at the sequence position corresponding to the last position. A part can also be defined by referring to a position in a specified polynucleotide sequence and the length of residues relative to the reference position, and thus the sequence of the part is a continuous nucleotide sequence in the specified polynucleotide, with a defined length and positioned in the specified polynucleotide according to the defined position.

In the present application, the term "modification of nucleotides" can mean the synthesis or modification of nucleic acids by mature methods in the field, such as those described in "Current protocols in nucleic acid chemistry" Beaucage, S.L. et al., (Edrs.), John Wiley&Sons, Inc., New York, NY, USA (incorporated herein by reference). The modification can include but is not limited to terminal modifications, such as 5'-terminal modifications (e.g., phosphorylation, conjugation, inverted linkage) or 3'-terminal modifications (e.g., conjugation, DNA nucleotides, inverted linkage, etc.); base modifications, such as substitutions with stabilized bases, destabilized bases, or bases that pair with an extended pairing group library base, base removal (nucleotides without base), or conjugated bases; sugar modifications (e.g., sugar modifications at the 2'-position or 4'-position) or sugar substitutions; or backbone modifications, including modifications or substitutions of phosphodiester bond.

In the present application, the term "substance that modifies histones" generally refers to related enzymes that can modify histones and regulate gene transcription. Common modifications to histones can be methylation, acetylation, phosphorylation, adenylation, ubiquitination, ADP-ribosylation, etc.

In the present application, the term "methylation modification" is used interchangeably with "DNA methylation" and "nucleic acid methylation", which generally refers to the methylation status of gene fragment, nucleotide, or their bases in the present application. This status often occurs within cells that have been transfected with nucleic acids, and the cells have been transfected with nucleic acids comprising structural genes encoding polypeptide effectively linked to promoters. In this process, the cytosine of promoter nucleic acid is converted into 5-methylcytosine. Promoter nucleic acids with at least one cytosine converted into 5-methylcytosine are referred to as "methylated" nucleic acids or DNA. The DNA fragments in which genes are located in the present application can have methylation on one strand or multiple strands, and can also have methylation at one site or multiple sites.

In the present application, the term "regulatory element" refers to a genetic element which can control the expression of nucleic acid sequences. For example, splicing signals, promoter sequences, polyadenylation signals, transcription termination sequences, upstream regulatory domains, replication origins, internal ribosome entry sites ("IRES"), enhancers, etc., together provide the replication, transcription, and translation of the encoding sequence in the recipient cell. Not all of these control sequences are required. Transcriptional control signals in eukaryotes typically include "promoter" and "enhancer" elements. Promoters and enhancers are composed of short arrays of DNA sequences; promoters are regulatory elements that facilitate the initiation of transcription of the operatively linked encoding region, while enhancers are regulatory elements that increase the activity of the nearest promoter on the same DNA molecule, thereby increasing the rate of genetic transcription. These sequences specifically interact with cellular proteins involved in transcription (Maniatis et al., Science 236: 1237 (1987), incorporated herein by reference in its entirety). Promoter and enhancer elements can be isolated from various eukaryotic sources, including genes in yeast, insect, and mammalian cells, as well as viruses (similar control sequences, i.e., promoters, are also found in prokaryotes). The choice of specific promoters and enhancers depends on the type of recipient cell. Some eukaryotic promoters and enhancers have a broad host range, while others function in a limited subgroup of cell types (for a review, see, for example, Voss et al., Trends Biochem. Sci., 11: 287 (1986); and Maniatis *et al.* (ibid), incorporated herein by reference in their entirety). For example, the SV40 early gene enhancer is very active in a variety of cell types from many mammalian species and has been used to express proteins in various mammalian cells (Dijkema et al., EMBO J.4: 761 (1985), incorporated herein by reference in its entirety). Promoter and enhancer elements derived from human elongation factor 1-α gene (Uetsuki et al., J. Biol. Chem., 264: 5791 (1989); Kim et al., Gene 91: 217 (1990); and Mizushima and Nagata, Nucl. Acids Res., 18: 5322 (1990)), long terminal repeat sequence of Rous sarcoma virus (Gorman et al., Proc. Natl. Acad. Sci. U.S.A. 79: 6777 (1982)) and human cytomegalovirus (Boshart et al., Cell 41: 521 (1985)) can also be used to express proteins in different mammalian cell types, the references cited herein are incorporated herein by reference in their entirety. Promoters and enhancers can exist naturally alone or together. For example, the long terminal repeat sequence of retroviruses contains both promoter and enhancer elements. Generally, promoters and enhancers act independently of the gene being transcribed or translated. Therefore, the enhancers and promoters used can be "endogenous", "exogenous", or "heterologous" relative to the gene they are operatively linked to. "Endogenous" enhancers/promoters are enhancers/promoters that are naturally linked to a given gene in the genome. "Exogenous" or "heterologous" enhancers or promoters are enhancers or promoters that are juxtaposed with a gene through genetic manipulation (i.e., molecular biology techniques), such that the transcription of the gene is directed by the linked enhancers/promoters. The presence of "splicing signal" on the expression vector usually results in high-level expression of recombinant transcripts. In some embodiments, "splicing signal" mediates the removal of introns from primary RNA transcripts, and composed of splicing donor and acceptor sites (Sambrook et al., Molecular Cloning: A Laboratory Manual, 2nd Edition, Cold Spring Harbor Laboratory Press, New York (1989), pp. 16.7-16.8, incorporated herein by reference in its entirety). Commonly used splicing donor and acceptor sites are the splice sites from the 16S RNA of SV40. In some embodiments, "transcription termination signals" are usually located downstream of the polyadenylation signal and are several hundreds of nucleotides in length. For example, the term "poly A signal" or "poly A sequence" refers to the DNA sequence that guides the termination and polyadenylation of the newly synthesized RNA transcript. Efficient polyadenylation of recombinant transcripts is often necessary because transcripts lacking the poly A signal are unstable and are rapidly degraded. The poly A signal used in the expression vector can be "heterologous" or "endogenous". Endogenous poly A signals are signals naturally present at the 3'-end of the coding region of a given gene in the genome. Heterologous poly A signals are signals that are isolated from one gene and operably linked to the 3'-end of another gene. A commonly used heterologous poly A signal is the SV40 poly A signal. The SV40 poly A signal is included in a 237 bp BamHI/BclI restriction fragment and guides termination and polyadenylation (Sambrook *et al.,* ibid, 16.6-16.7, incorporated herein by reference).

In the present application, the term "DNA methyltransferase" generally refers to an enzyme that catalyzes the transfer of a methyl to DNA. Non-limiting examples of DNA methyltransferases include DNMT1, DNMT 3A, DNMT 3B, and DNMT 3L. For example, through DNA methylation, DNA methyltransferases can modify the activity of DNA fragments (such as regulating gene expression) without changing the DNA sequence. As described herein, gene expression-regulating molecules may include one or more (e.g., two) DNA methyltransferases. When DNA methyltransferases are included as part of a gene expression-regulating molecule, they can be referred to as a "DNA methyltransferase domain". In all aspects, the DNA methyltransferase domain includes variants or homologs with an amino acid sequence that has at least 50%, at least 55%, at least 60%, at least 65%, at least 70%, at least 75%, at least 80%, at least 85%, 90%, at least 91%, at least 92%, at least 93%, at least 94%, at least 95%, at least 96%, at least 97%, at least 98%, at least 99%, or 100% sequence identity with DNMT 3A. In all aspects, the DNA methyltransferase domain includes variants or homologs with an amino acid sequence that has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity with DNMT 3L.

In the present application, the term "functional active fragment" generally refers to a fragment that has a partial region of a full-length protein or nucleic acid, and retains or partially retains the biological activity or function of the full-length protein or nucleic acid. For example, a functional active fragment can retain or partially retain the ability of the full-length protein to bind to another molecule. For example, a functional active fragment of a DNA methyltransferase can retain or partially retain the biological activity function of the full-length DNA methyltransferase to catalyze the transfer of a methyl to DNA.

In the present application, the term "direct and/or indirect link" generally refers to the relatively "direct link" or "indirect link". The term "direct link" generally refers to a direct connection or binding. For example, the direct link can be a situation where there are no spacer components (such as amino acid residues or their derivatives) between the connected materials (e.g., amino acid sequence segments) that are directly connected; for example, an amino acid sequence segment X is directly linked to another amino acid sequence segment Y through an amide bond formed by the C-terminal amino acid of the amino acid sequence segment X and the N-terminal amino acid of the amino acid sequence segment Y. "Indirect link" generally refers to a situation where there are spacer components (such as amino acid residues or their derivatives) between the connected materials (e.g., amino acid sequence segments) that are indirectly linked.

In the present application, the term "Krab", also known as "Kruppel-associated box domain" or "Krüppel-associated box domain", generally refers to about 45 to about 75 amino acid residues present in a transcriptional repression domain of a human zinc finger transcription factor. In all aspects, the Krab domain can include variants or homologs with an amino acid sequence that has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity with the ZIM3 Krab domain or KOX1 Krab domain.

In the present application, the term "delivery carrier" generally refers to a transfer vehicle that can deliver reagents (e.g., nucleic acid molecules) to target cells. The delivery carrier can deliver reagents to specific subclasses of cells. For example, the delivery carrier can target certain types of cells by virtue of its inherent characteristics or through parts conjugated to the carrier, parts contained within it (or parts combined with the carrier, thereby keeping the part and the delivery carrier together, and thus making the part sufficient to target the delivery carrier). The delivery carrier can also increase the *in vivo* half-life and/or bioavailability of the reagents to be delivered. The delivery carrier can include viral vectors, virus-like particles, polycationic carriers, peptide carriers, liposomes, and/or hybrid carriers. For example, if the target cells are hepatocytes, the properties of the delivery carrier (e.g., size, charge, and/or pH) can effectively deliver the delivery carrier and/or the molecules it carries to the target cells, reduce immune clearance, and/or promote residence in the target cells.

In the present application, the term "liposome" generally refers to a vesicle with an internal space isolated from the external medium by one or more bilayers. In some embodiments, the bilayer can be formed by amphiphilic molecules, such as synthetic or naturally derived lipids that include hydrophilic and hydrophobic structural domains with spatial isolation; in other embodiments, the bilayer can be formed by amphiphilic polymers and surfactants. In some embodiments, the liposome is a spherical vesicle structure composed of a single or multilayer lipid bilayer surrounding an internal aqueous compartment and a relatively impermeable external lipophilic phospholipid bilayer. In some embodiments, liposomes are biocompatible and non-toxic, can deliver hydrophilic and lipophilic drug molecules, protect their cargo from degradation by plasma enzymes, and transport their load across biological membranes and the blood-brain barrier (BBB). Liposomes can be made from several types of lipids such as phospholipids. Liposomes can contain natural phospholipids and lipids (such as 1,2-distearoyl-sn-glycero-3-phosphocholine (DSPC), sphingomyelin, phosphatidylcholine, monosialoganglioside, or any combination thereof. To alter the structure and properties of liposomes, several other additives can be added in the liposomes. For example, liposomes can also contain cholesterol, sphingomyelin, and/or 1,2-dioleoyl-sn-glycero-3-phosphoethanolamine (DOPE), for example, to increase stability and/or prevent leakage of the cargo within the liposomes.

The term "lipid nanoparticle (LNP)" generally refers to particles that comprise multiple (i.e., more than one) lipid molecules physically bound to each other through intermolecular forces (e.g., covalent or non-covalent). LNPs can be, for example, microspheres (including single-layer and multilayer vesicles, such as liposomes), dispersed phases in emulsions, micelles, or internal phases in suspensions. LNPs can encapsulate nucleic acids within cationic lipid particles (e.g., liposomes) and can be relatively easily delivered to cells. In some examples, lipid nanoparticles do not contain any viral components, which helps to minimize safety and immunogenicity issues. The lipid particles can be used for *in vitro, ex vivo,* and *in vivo* delivery. The lipid particles can also be used for various scales of cell populations. The LNPs of the present application can be easily prepared by various methods known in the field, such as by mixing an organic phase with an aqueous phase. The mixing of the two phases can be achieved through microfluidic devices and impinging stream reactors. The more thorough the mixing of the organic and aqueous phases, the better the encapsulation efficiency and particle size distribution of the resulting LNPs. Preferably, the particle size of the LNPs can be adjusted by changing the mixing speed of the organic and aqueous phases. The faster the mixing speed, the smaller the particle size of the prepared LNPs. The encapsulation efficiency can be optimized by adjusting the N/P (ionizable lipid/nucleic acid) ratio of the LNP system. In some examples, LNPs can be used to deliver DNA molecules (e.g., molecules comprising encoding sequences of DNA-binding proteins and/or sgRNA) and/or RNA molecules (e.g., mRNA of Cas, mRNA of sgRNA). In certain cases, LNPs can be used to deliver Cas/sgRNA RNP complexes. In some embodiments, LNPs are used to deliver mRNA and gRNA (e.g., mRNA fusion molecule comprising DNMT3A-DNMT3L(3A-3L)-dCas9-KRAB and at least one sgRNA targeting a target gene).

In the present application, the term "recombinant vector" generally refers to a nucleic acid molecule that can transport itself and another nucleic acid connected to it. One type of vector is a "plasmid", which refers to a circular double-stranded DNA loop to which additional DNA segments can be linked internally. Alternatively, the vector can be linear. Another type of vector is a viral vector, in which additional DNA segments can be linked to the viral genome. Specific vectors can replicate autonomously within the host cells they are introduced to (e.g., bacterial vectors with bacterial replication origins and episomal mammalian vectors). Other vectors (e.g., non-episomal mammalian vectors) can integrate into the host cell genome after being introduced to the host cell and thus replicate along with the host genome.

In the present application, the term "adeno-associated virus (AAV) vector" generally refers to a vector with functional or partially functional ITR sequences and a transgene. As used herein, the term "ITR" refers to inverted terminal repeat sequences. ITR sequences can be derived from adeno-associated virus serotypes, including but not limited to AAV-1, AAV-2, AAV-3, AAV-4, AAV-5, AAV-6 6, AAV7, AAV8, AAV9, AAV10, AAV11, AAV12, and AAV13, as well as any AAV variants or mixtures. However, ITRs need not be wild-type nucleotide sequences and can be altered (e.g., through insertion, deletion, or substitution of nucleotides) as long as the sequence retains the function of providing functional rescue, replication, and packaging. AAV vectors may have one or more entirely or partially deleted AAV wild-type genes, preferably rep and/or cap genes, but retain functional flanking ITR sequences. Functional ITR sequences serve, for example, to rescue, replicate, and package AAV virus particles or granule. Therefore, an "AAV vector" is defined in the present application as including at least those sequences required to insert a transgene into a subject's cells. Optionally, it includes those cis-sequences necessary for virus replication and packaging (e.g., functional ITR).

In the present application, the terms "nuclear localization sequence" or "nuclear localization signal" or "NLS" generally refer to peptides that direct proteins to the cell nucleus. For example, NLS includes five basic and positively charged amino acids. For example, NLS can be located at any position on the peptide chain.

In the present application, the terms "complementary" or "complementarity" generally refer to the ability of nucleic acids to form hydrogen bonds with another nucleic acid sequence through traditional Watson-Crick or other non-traditional types. For example, the sequence A-G-T is complementary to the sequence T-C-A. The percentage of complementarity indicates the percentage of residues in a nucleic acid molecule that can form hydrogen bonds (e.g., Watson-Crick base pairing) with a second nucleic acid sequence (e.g., five out of ten, six out of ten, seven out of ten, eight out of ten, nine out of ten, ten out of ten represent 50%, 60%, 70%, 80%, 90%, and 100% complementarity, respectively). For example, "fully complementary" means that all consecutive residues of a nucleic acid sequence will hydrogen bond with the same number of consecutive residues in a second nucleic acid sequence. For example, "substantially complementary" means that two nucleic acids have at least 60%, 65%, 70%, 75%, 80%, 85%, 90%, 95%, 97%, 98%, 99%, or 100% complementarity over an area of 8, 9, 10, 11, 12, 13, 14, 15, 16, 17, 18, 19, 20, 21, 22, 23, 24, 25, 30, 35, 40, 45, 50 or more nucleotides, or refer to two nucleic acids that hybridize under strict conditions (i.e., strict hybridization conditions) with at least 60%, 65%, 70%, 75%, 80%, 85%, 90%, 95%, 97%, 98%, 99%, or 100% complementarity.

In the present application, the term "gene" generally refers to a DNA fragment designed to produce a protein. For example, the gene can also include regions before and after the coding region (leader and trailer) as well as intervening sequence (intron) between each coding fragment (exon). Leaders, trailers, and introns include regulatory elements necessary for gene transcription and translation processes. In addition, the term "protein gene product" can refer to a protein expressed by a specific gene.

In the present application, the terms "polypeptide", "peptide", and "protein" are used interchangeably to refer to polymers of amino acid residues. For example, the polymer can combine with parts that are not composed of amino acids in all aspects. For example, a "fusion protein" can refer to a chimeric protein of two or more separate protein sequences each encoded and expressed in a recombinant manner as an individual part. In the case of two or more nucleic acid or polypeptide sequences, the terms "identical" or percentage "identity" refer to the measurement obtained using the BLAST or BLAST 2.0 sequence alignment algorithm with the default parameters as described below or through manual alignment and visual inspection. For example, two or more sequences or subsequences with identical or specified percentage of identical amino acid residues or nucleotides (when compared and aligned for maximum correspondence in a comparison window or specified area, with about 60% identity, preferably 65%, 70%, 75%, 80%, 85%, 90%, 91%, 92%, 93%, 94%, 95%, 96%, 97%, 98%, 99%, or higher identity) can be referred to as "substantially identical".

In the present application, the term "guide RNA" or "gRNA" generally refers to any polynucleotide sequence that has sufficient complementarity with a target polynucleotide sequence to hybridize with the target sequence, and specifically bind the CRISPR complex to the target sequence. In all aspects, the degree of complementarity between the guide sequence and its corresponding target sequence is about or greater than about 50%, about 60%, about 75%, about 80%, about 85%, about 90%, about 95%, about 97.5%, about 99%, or higher when optimally aligned using a suitable alignment algorithm.

In the present application, the specific proteins (e.g., KRAB, dCas9, Dnmt3A, Dnmt3L) can include any natural form of the protein or variants or homologs that maintain the activity of the protein (e.g., having at least 50%, at least 60%, at least 70%, at least 80%, at least 90%, at least 95%, at least 96%, at least 97%, at least 98%, at least 99%, or 100% of the activity compared to the natural protein). In all aspects, variants or homologs have at least 50%, at least 55%, at least 60%, at least 65%, at least 70%, at least 75%, at least 80%, at least 85%, at least 90%, at least 95%, at least 96%, at least 97%, at least 98%, at least 99%, or 100% amino acid sequence identity over the entire sequence or a part of the sequence (e.g., 50, 100, 150, or 200 consecutive amino acid parts), compared to the naturally occurring form.

In the present application, the term "linker" generally refers to a connector that connects two or more parts. In each embodiment, linkers are attached at the N-terminal and C-terminal to the amino acid sequences of the compound's remaining parts (e.g., the fusion proteins provided herein). For example, the terms "XTEN", "XTEN linker", or "XTEN polypeptide" refer to recombinant polypeptides that lack hydrophobic amino acid residues.

In the present application, the terms "detectable agent" or "detectable portion" refer to compositions that can be detected by appropriate means. For example, the methods can be spectroscopic, photochemical, biochemical, immunochemical, chemical, magnetic resonance imaging, or other physical means. For example, useful detectable reagents include radioactive elements, fluorophores (e.g., fluorescent dyes), electron-dense reagents, enzymes (e.g., commonly used in ELISA), biotin, paramagnetic molecules, etc.

In the present application, the terms "inhibition", "repression", "silencing", and the like generally refer to the reduction of gene expression and/or activity. For example, the administration of substance (e.g., fusion protein, complex, nucleic acid, vector) in the present application can have a negative impact (e.g., reduction) on the activity of a nucleic acid sequence relative to the activity in the absence of the substance (control). For example, inhibition can refer to a reduction in disease or disease symptoms. For example, inhibition includes at least partially, partially, or completely blocking the activation of a nucleic acid sequence (e.g., transcription), or reducing, preventing, or delaying the activation of a nucleic acid sequence. For example, inhibitory activity can be 90%, 80%, 70%, 60%, 50%, 40%, 30%, 20%, 10%, or lower than the control.

In the present application, the term "comprising" generally means including the specifically designated features but does not exclude other elements.

In the present application, the term "selected from" generally means including the selected object and all its combinations. For example, "selected from (:) A, B, and C" means including all combinations of A, B, and C, such as A, B, C, A+B, A+C, B+C, or A+B+C.

In the present application, the term "about" generally refers to a variation within a range of 0.5%-10% above or below the specified value, for example, a variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

### DETAILED DESCRIPTION OF THE INVENTION

In an aspect, the present application provides a method for regulating the expression and/or activity of the LPA gene, comprising providing a gene expression-regulating molecule or a nucleic acid encoding the gene expression-regulating molecule, the gene expression-regulating molecule may have the function of regulating the expression of the LPA gene without altering its gene sequence. For example, the method of the present application can be a method not intended for therapeutic purposes. For example, the method of the present application can be a method not directly targeting the human body. For example, the method of the present application can be an *in vitro* or *ex vivo* method. For example, the method of the present application can be a method intended for therapeutic purposes. For example, the method of the present application can be an *in vivo* method.

In another aspect, the present application provides a method for treating and/or alleviating a condition related to abnormal expression and/or activity of the LPA gene, comprising providing a gene expression-regulating molecule or a nucleic acid encoding the gene expression-regulating molecule, the gene expression-regulating molecule may have the function of regulating the expression of the LPA gene without altering its gene sequence.

In another aspect, the present application provides a gene expression-regulating molecule or a nucleic acid encoding the gene expression-regulating molecule, wherein the gene expression-regulating molecule may have the function of regulating the expression of the LPA gene without altering its gene sequence. For example, the nucleic acid comprises DNA and/or mRNA. For example, the gene expression-regulating molecule or the nucleic acid encoding the gene expression-regulating molecule may be used for treating and/or alleviating a condition related to abnormal expression and/or activity of the LPA gene.

In another aspect, the present application provides use of a gene expression-regulating molecule or a nucleic acid encoding the gene expression-regulating molecule in the preparation of a medicament for treating and/or alleviating a condition related to abnormal expression and/or activity of the LPA gene, the gene expression-regulating molecule may have the function of regulating the expression of the LPA gene without altering its gene sequence. For example, the nucleic acid encoding the gene expression-regulating molecule of the present application comprises DNA and/or mRNA.

In another aspect, the present application provides a nucleic acid-binding molecule or a nucleic acid encoding the nucleic acid-binding molecule, the nucleic acid-binding molecule comprising a sequence as shown in any one of SEQ ID NOs: 1-50. For example, the nucleic acid encoding the nucleic acid-binding molecule of the present application comprises DNA and/or mRNA.

In another aspect, the present application provides a recombinant vector comprising the nucleic acid of the present application. For example, the recombinant vector may refer to a nucleic acid molecule capable of transporting another nucleic acid molecule connected with it. The recombinant vector may include single-stranded, double-stranded, or partially double-stranded nucleic acid molecules; nucleic acid molecules with one or more free ends, without free ends (e.g., loop); nucleic acid molecules comprising DNA, RNA, or both; and other types of polynucleotides known in the field. For example, viral vectors may be used. Viral vectors may comprise viral-derived DNA or RNA sequences for packaging into viruses (e.g., retroviruses, replication-defective retroviruses, adenoviruses, replication-defective adenoviruses, and adeno-associated virus AAV). Viruses and viral vectors may be used for *in vitro, ex vivo,* and/or *in vivo* delivery.

In another aspect, the present application provides a delivery carrier comprising the nucleic acid-binding molecule of the present application, the gene expression-regulating molecule of the present application, the nucleic acid of the present application, and/or the recombinant vector of the present application, and optionally comprising liposomes and/or lipid nanoparticles (LNP). For example, the delivery carrier may comprise one or more Cas proteins and one or more guide RNAs, for example, in the form of a ribonucleoprotein complex (RNP). For example, ribonucleoproteins may be delivered via polypeptide-based shuttle agents. For example, ribonucleoproteins may be delivered using synthetic peptides. For example, the delivery carrier may be introduced into cells via physical delivery methods. Examples of physical methods include microinjection, electroporation, and hydrodynamic delivery. For example, LNPs may encapsulate nucleic acids in cationic lipid particles (e.g., liposomes) and can be relatively easily delivered to cells. In some examples, lipid nanoparticles do not contain any viral components, which helps to minimize safety and immunogenicity issues. Lipid particles may be used for *in vitro, ex vivo,* and *in vivo* delivery. The components of LNPs may include cationic lipids, ionizable lipids, PEGylated lipids, and/or helper lipids, as well as optional cholesterol components. In some embodiments, LNPs may comprise ionizable lipids (20%-70%, molar ratio), PEGylated lipids (0%-30%, molar ratio), helper lipids (30%-50%, molar ratio), and cholesterol (10%-50%, molar ratio).

In another aspect, the present application provides a composition comprising the nucleic acid-binding molecule of the present application, the gene expression-regulating molecule of the present application, the nucleic acid of the present application, the recombinant vector of the present application, and/or the delivery carrier of the present application. For example, the nucleic acid-binding molecule, gene expression-regulating molecule, nucleic acid encoding the nucleic acid-binding molecule, nucleic acid encoding the gene expression-regulating molecule, recombinant vector, and delivery carrier in the composition may be included in one composition or included in different compositions. For example, when using the nucleic acid-binding molecule, gene expression-regulating molecule, nucleic acid encoding the nucleic acid-binding molecule, nucleic acid encoding the gene expression-regulating molecule, recombinant vector, and/or delivery carrier in the composition, they may be used simultaneously or separately.

In another aspect, the present application provides a cell comprising the nucleic acid-binding molecule of the present application, the gene expression-regulating molecule of the present application, the nucleic acid of the present application, the recombinant vector of the present application, the delivery carrier of the present application, and/or the composition of the present application.

In another aspect, the present application provides a kit comprising the nucleic acid-binding molecule of the present application, the gene expression-regulating molecule of the present application, the nucleic acid of the present application, the recombinant vector of the present application, the delivery carrier of the present application, the composition of the present application, and/or the cell of the present application.

In another aspect, the present application provides a method for regulating the expression and/or activity of a target gene, comprising providing the nucleic acid-binding molecule of the present application, the gene expression-regulating molecule of the present application, the nucleic acid of the present application, the recombinant vector of the present application, the delivery carrier of the present application, the composition of the present application, the cell of the present application, and/or the kit of the present application. For example, the method may reduce the expression and/or activity of the target gene. For example, compared to the expression and/or activity of the target gene in the absence of the substance of the present application, the administration of the substance of the present application may have a negative impact (e.g., reduction) on the activity of the nucleic acid sequence, which may include at least partially, partially, or completely blocking the activation of the nucleic acid sequence (e.g., transcription), or reducing, preventing, or delaying the activation of the nucleic acid sequence. For example, the inhibition of activity may be about 90%, about 80%, about 70%, about 60%, about 50%, about 40%, about 30%, about 20%, about 10%, or lower of the control.

In another aspect, the present application provides use of the nucleic acid-binding molecule of the present application, the gene expression-regulating molecule of the present application, the nucleic acid of the present application, the recombinant vector of the present application, the delivery carrier of the present application, the composition of the present application, the cell of the present application, and/or the kit of the present application in the preparation of a medicament for treating and/or alleviating a condition, said condition comprises a condition related to abnormal expression and/or activity of the target gene.

In another aspect, the present application provides the nucleic acid-binding molecule of the present application, the gene expression-regulating molecule of the present application, the nucleic acid of the present application, the recombinant vector of the present application, the delivery carrier of the present application, the composition of the present application, the cell of the present application, and/or the kit of the present application for use in treating and/or alleviating a condition, said condition comprises a condition related to abnormal expression and/or activity of the target gene.

In another aspect, the present application provides a method for treating and/or alleviating a condition, comprising providing the nucleic acid-binding molecule of the present application, the gene expression-regulating molecule of the present application, the nucleic acid of the present application, the recombinant vector of the present application, the delivery carrier of the present application, the composition of the present application, the cell of the present application, and/or the kit of the present application, said condition comprises a condition related to abnormal expression and/or activity of the target gene. In some embodiments, the treatment targets organ diseases/disorders, exemplary of which may include liver diseases, eye diseases, muscle diseases, heart diseases, blood diseases, brain diseases, kidney diseases, or may include treatments for autoimmune diseases, central nervous system diseases, cancer and other proliferative diseases, neurodegenerative diseases, inflammatory diseases, metabolic diseases, musculoskeletal diseases, etc.

### Gene expression-regulating molecules

The gene expression molecules provided in the present application or in the methods of the present application has the function of regulating the expression of the LPA gene without altering its gene sequence. For example, the gene expression-regulating molecule may have the function of inhibiting gene expression.

In some embodiments, the gene expression-regulating molecule comprises a first functional domain, the first functional domain provides modification of at least one nucleotide in the vicinity of the LPA gene and/or within the LPA gene regulatory element. In some embodiments, the first functional domain may regulate the expression of the target gene at the regulatory elements of the target gene, such as promoters, enhancers, or transcription start sites, through epigenetic modifications, such as DNA methylation. For example, regulatory elements may include transcription start sites, core promoters, proximal promoters, distal enhancers, silencers, insulator elements, boundary elements, or locus control regions. For example, the epigenetic modification may be carried out by any known epigenetic modifier that can be used for DNA methylation, and exemplary epigenetic modifiers may include DNA methyltransferases (e.g., DNMT3A or DNMT3A-DNMT3L), DNA demethylases (e.g., TET1 catalytic domain or TDG), and/or their functional active fragments.

For example, the first functional domain may provide modification of at least one nucleotide in the vicinity of a LPA gene and/or within a LPA gene regulatory element, the modification of at least one nucleotide comprises methylation modification. In some embodiments, the first functional domain comprises an epigenetic modifier that may have DNA methylase activity. For example, the epigenetic modifier may have methylase activity, which involves the transfer of a methyl group to DNA, RNA, proteins, small molecules, cytosine, or adenine. For example, the modification may be located within about 100 bp, about 200 bp, about 300 bp, about 400 bp, about 500 bp, about 600 bp, about 700 bp, about 800 bp, about 900 bp, about 1000 bp, about 1100 bp, about 1200 bp, about 1300 bp, about 1400 bp, or about 1500 bp upstream of the transcription start site of the target gene. For example, the modification may be located within about 100 bp, about 200 bp, about 300 bp, about 400 bp, about 500 bp, about 600 bp, about 700 bp, about 800 bp, about 900 bp, about 1000 bp, about 1100 bp, about 1200 bp, about 1300 bp, about 1400 bp, or about 1500 bp downstream of the transcription start site of the target gene.

For example, the first functional domain may comprise a DNA methyltransferase (DNMT) domain. For example, the first functional domain may comprise a DNMT 3A domain and/or a DNMT 3L domain. For example, the DNMT 3A domain and/or DNMT 3L domain are derived from mammals. For example, the DNMT 3A domain is derived from mice. For example, the gene expression-regulating molecule may comprise an amino acid sequence of DNMT 3A. For example, the DNMT 3L domain is derived from human and/or mouse. For example, the gene expression-regulating molecule may comprise an amino acid sequence of DNMT 3L. Alternatively, it may have one, two, three, four, five, or more changes compared to the above sequences, such as sequences with amino acid substitutions, insertions, or deletions, or any fragments thereof.

For example, the DNMT 3A domain and the DNMT 3L domain may be directly and/or indirectly linked. For example, the DNMT 3A domain and the DNMT 3L domain may be linked via a linker. For example, the C-terminal of the DNMT 3A domain may be linked to the N-terminal of the DNMT 3L, or the C-terminal of the DNMT 3L domain may be linked to the N-terminal of the DNMT 3A.

In some embodiments, the gene expression-regulating molecule comprises a second functional domain, the second functional domain comprises a transcription factor based on zinc finger proteins or a functional active fragment thereof, or a substance capable of modifying histones. For example, the second functional domain may comprise a gene expression repressor, which may be any known gene expression repressor, and exemplary gene expression repressors may be selected from the Krüppel-associated box (KRAB) domain, mSin3 interaction domain (SID), MAX interaction protein 1 (MXI1), chromo shadow domain, EAR-repression domain (SRDX), eukaryotic release factor 1 (ERF1), eukaryotic release factor 3 (ERF3), tetracycline repressor, lad repressor, Catharanthus roseus G-box binding factor 1 and 2, Drosophila Groucho (drosophila Gro protein), tripartite motif-containing 28 (TRTM28), nuclear receptor corepressor 1, nuclear receptor corepressor 2, or their functional active fragments or fusions. For example, the second functional domain may comprise a substance capable of modifying histones. In some embodiments, the second functional domain may comprise a Krab domain. Specifically, the second functional domain may comprise a ZIM3 Krab domain or a KOX1 Krab domain.

In some embodiments, the KRAB domain or a fragment thereof may be fused to the C-terminal of the dCas9 molecule. In some embodiments, the KRAB domain or a fragment thereof may be fused to both the N-terminal and C-terminal of the dCas9 molecule. In some embodiments, the second functional domain may comprise a KRAB domain, which may comprise a sequence that is substantially identical to (e.g., at least 80%, at least 85%, at least 90%, at least 91%, at least 92%, at least 93%, at least 94%, at least 95%, at least 96%, at least 97%, at least 98%, at least 99%, or higher identity) to ZIM3 Krab or KOX1 Krab, or a sequence with one, two, three, four, five, or more changes (e.g., amino acid substitutions, insertions, or deletions relative to ZIM3 Krab or KOX1 Krab), or any fragment thereof.

In some embodiments, the second functional domain may comprise a gene expression activator, which may be any known gene expression activator, and exemplary gene expression activators may include VP16 activation domain, VP64 activation domain, p65 activation domain, Epstein-Barr virus R transactivation factor Rta molecule or a fragment thereof.

In some embodiments, the second functional domain may comprise a substance capable of modifying histones, which may include histone acetyltransferases (e.g., p300 catalytic domain), histone deacetylases, histone methyltransferases (e.g., SUV39H1 or G9a (EHMT2)), histone demethylases (e.g., LSD1), and/or their functional active fragments.

In some of the above cases, the gene expression-regulating molecule comprises the first functional domain and the second functional domain, and the first functional domain is directly or indirectly linked to one end of the second functional domain, or the first functional domain is directly and/or indirectly linked to both ends of the second functional domain. For example, the C-terminal of the first functional domain is directly linked to the N-terminal of the second functional domain, or the C-terminal of the first functional domain is indirectly linked (e.g., via a linker) to the N-terminal of the second functional domain, or the first functional domain is located on the N-terminal side of the second functional domain (e.g., the order from N-terminal to C-terminal may be first functional domain, other parts such as DNA binding domain, and second functional domain). For example, the C-terminal of the second functional domain is directly linked to the N-terminal of the first functional domain, or the C-terminal of the second functional domain is indirectly linked (e.g., via a linker) to the N-terminal of the first functional domain, or the second functional domain is located on the N-terminal side of the first functional domain (e.g., the order from N-terminal to C-terminal may be second functional domain, other parts such as DNA binding domain, and first functional domain). For example, the first functional domain comprises two or more functional domains, each of which may be directly linked to, indirectly linked to (e.g., via a linker), or located on the N-terminal side and C-terminal side of the second functional domain (e.g., the order from N-terminal to C-terminal may be first functional domain a, other parts such as DNA binding domain, second functional domain, and first functional domain b, or may be first functional domain a, second functional domain, other parts such as DNA binding domain, and first functional domain b, wherein a and b are different types of first functional domains).

In some embodiments, the gene expression-regulating molecule comprises a DNA binding domain. For example, the gene expression-regulating molecule may have the function of binding to gene sequences. For example, the DNA binding domain comprises a (DNA) nuclease, which may be a nuclease that targets DNA in a sequence-specific manner; exemplary nucleases may be CRISPR-Cas system-related enzymes, zinc finger nucleases (ZFN), transcription activator-like effector nucleases (TALEN), or meganucleases. For example, the gene expression-regulating molecule may comprise the DNA binding domain of TALEN, zinc finger domain, and/or DNA binding domain of a CRISPR/Cas system. In some cases, the DNA binding domain is a DNA nuclease derived from the CRISPR-Cas system. In some cases, the DNA binding domain is a (modified) transcription activator-like effector nuclease (TALEN) system; the transcription activator-like effector (TALE) may be designed to bind almost any desired DNA sequence. In some cases, the DNA binding domain is a (modified) zinc finger nuclease (ZFN) system or composed thereof; the ZFN system uses artificial restriction enzymes produced by fusing zinc finger DNA binding domains with DNA cleavage domains, the DNA cleavage domains can be engineered to target desired DNA sequences. In some cases, the DNA binding domain is a (modified) meganuclease, which is an endodeoxyribonuclease characterized by a large recognition site (12 to 40 base pair double-stranded DNA sequences).

In some embodiments, the gene expression-regulating molecule may comprise a Cas enzyme. For example, the gene expression-regulating molecule may comprise a Cas enzyme that substantially lacks nuclease activity. Typically, the guide sequence (or spacer sequence) may be any polynucleotide sequence that has sufficient complementarity with the target polynucleotide sequence to hybridize with the target sequence and guide the CRISPR complex to bind to the target sequence in a sequence-specific manner. In some cases, the degree of complementarity between the guide sequence and its corresponding target sequence may be about or greater than about 50%, 60%, 70%, 75%, 80%, 85%, 90%, 91%, 92%, 93%, 94%, 95%, 96%, 97%, 98%, 99%, or more when the best match is performed using a suitable alignment algorithm. In some embodiments, the Cas protein targeting nucleic acids may be mutated relative to the corresponding wild-type enzyme, such that the mutated Cas protein targeting nucleic acids lacks the ability to cleave one or both strands of the target polynucleotide. For example, the DNA cleavage activity of the mutated enzyme may be no more than 25%, 10%, 5%, 1%, 0.1%, 0.01%, or less of that of the non-mutated form of the enzyme. In some embodiments, the mutated Cas may have one or more mutations, resulting in reduced off-target effects.

For example, the gene expression-regulating molecule may comprise a Cas9 enzyme. Examples of Cas proteins include Class I (e.g., Type I, III, and IV) and Class 2 (e.g., Type II, V, and VI) Cas proteins, such as Cas9, Cas12 (e.g., Cas12a, Cas12b, Cas12c, Cas12d), Cas13 (e.g., Cas13a, Cas13b, Cas13c, Cas13d), CasX, CasY, Cas14, their variants (e.g., mutated forms, truncated forms), their homologs, and their orthologs. In some embodiments, the Cas protein is Cas9, Cas12a, Cas12b, Cas12c, or Cas12d. In some examples, Cas9 may be SpCas9, SaCas9, StCas9, and other Cas9 orthologs. Cas12 may be Cas12a, Cas12b, and Cas12c, including FnCas12a, or its homologs or orthologs. For example, the Cas9 enzyme may comprise *Staphylococcus aureus* dCas9, *Streptococcus pyogenes* dCas9, *Campylobacter jejuni* dCas9, *Corynebacterium diphtheria* dCas9, *Eubacterium ventriosum* dCas9, *Streptococcus pasteurianus* dCas9, *Lactobacillus farciminis* dCas9, *Sphaerochaeta globus* dCas9, *Azospirillum* (e.g., B510) dCas9, *Gluconacetobacter diazotrophicus* dCas9, *Neisseria cinerea* dCas9, *Roseburia intestinalis* dCas9, *Parvibaculum lavamentivorans* dCas9, *Nitratifractor salsuginis* (e.g., DSM 16511) dCas9, *Campylobacter lari* (e.g., CF89-12) dCas9, and/or *Streptococcus thermophilus* (e.g., strain LMD-9) dCas9. For example, the gene expression-regulating molecule may comprise the amino acid sequence of dCas9. Alternatively, it may have one, two, three, four, five, or more changes compared to the above dCas9 sequence, such as sequences with amino acid substitutions, insertions, or deletions, or any fragments thereof.

In some embodiments, the first functional domain and the second functional domain are directly or indirectly linked to one end of the DNA binding domain. For example, the first functional domain and the second functional domain are directly or indirectly linked to the C-terminal of the DNA binding domain, and exemplary gene-regulating molecules may comprise any one of dCas9-DNMT 3A-DNMT 3L-Krab, dCas9-DNMT 3L-DNMT 3A-Krab, dCas9-Krab-DNMT 3A-DNMT 3L, dCas9-Krab-DNMT 3L-DNMT 3A. For example, the first functional domain and the second functional domain are directly or indirectly linked to the N-terminal of the DNA binding domain, and exemplary gene-regulating molecules may comprise any one of DNMT 3A-DNMT 3L-Krab-dCas9, DNMT 3L-DNMT 3A-Krab-dCas9, Krab-DNMT 3A-DNMT 3L-dCas9, Krab-DNMT 3L-DNMT 3A-dCas9. In other embodiments, the first functional domain and the second functional domain are directly and/or indirectly linked to both ends of the DNA binding domain. For example, the first functional domain is directly or indirectly linked to the C-terminal of the DNA binding domain, and the second functional domain is directly or indirectly linked to the N-terminal of the DNA binding domain, and exemplary gene-regulating molecules may comprise any one of Krab-dCas9-DNMT 3A-DNMT 3L and Krab-dCas9-DNMT 3L-DNMT 3A. For example, the first functional domain is directly or indirectly linked to the N-terminal of the DNA binding domain, and the second functional domain is directly or indirectly linked to the C-terminal of the DNA binding domain, and exemplary gene-regulating molecules may comprise any one of DNMT 3A-DNMT 3L-dCas9-Krab and DNMT 3L-DNMT 3A-dCas9-Krab.

In some embodiments, the gene expression-regulating molecule is capable of binding to DNA region within about 500 bp upstream and/or downstream of the transcription start site (TSS) of the LPA genes or a fragment thereof. For example, the gene expression-regulating molecule is capable of binding to within about 100 bp, about 200 bp, about 300 bp, about 400 bp, about 500 bp, about 600 bp, about 700 bp, about 800 bp, about 900 bp, about 1000 bp, about 1100 bp, about 1200 bp, about 1300 bp, about 1400 bp, or about 1500 bp upstream of the TSS. For example, the gene expression-regulating molecule is capable of binding to within about 100 bp, about 200 bp, about 300 bp, about 400 bp, about 500 bp, about 600 bp, about 700 bp, about 800 bp, about 900 bp, about 1000 bp, about 1100 bp, about 1200 bp, about 1300 bp, about 1400 bp, or about 1500 bp downstream of the TSS. For example, the gene expression-regulating molecule is capable of binding to the DNA region where SEQ ID NO: 51 is located, or a fragment thereof.

Exemplarily, in some cases, the gene expression-regulating molecule is capable of binding to one or more DNA regions in the vicinity of the transcription start site (TSS) of the LPA gene as described below: between 410 bp upstream and 380 bp upstream of the TSS, between 230 bp upstream and 140 bp upstream of the TSS, and between 260 bp downstream and 370 bp downstream of the TSS.

In some embodiments, the gene expression-regulating molecule is capable of binding to one or more DNA regions in the vicinity of the transcription start site (TSS) of the LPA gene as described below: between 410 bp upstream and 380 bp upstream of the TSS (e.g., between 408-389 bp upstream of the TSS), between 230 bp upstream and 190 bp upstream of the TSS (e.g., between 222-203 bp upstream and/or 207-188 bp upstream of the TSS), between 170 bp upstream and 140 bp upstream of the TSS (e.g., between 163-144 bp upstream of the TSS), between 260 bp downstream and 290 bp downstream of the TSS (e.g., between 266-285 bp downstream of the TSS), and between 330 bp downstream and 370 bp downstream of the TSS (e.g., between 330-349 bp downstream and/or 344-363 bp downstream of the TSS).

In some embodiments, the gene expression-regulating molecule may also comprise a tag for detection, isolation, and/or purification. For example, the gene expression-regulating molecule may comprise an HA tag. For example, the gene expression-regulating molecule may comprise the amino acid sequence of the HA tag. Alternatively, it may have one, two, three, four, five, or more changes compared to the above sequence, such as sequences with amino acid substitutions, insertions, or deletions, or any fragments thereof.

In some embodiments, the gene expression-regulating molecule may also comprise a nuclear localization sequence. For example, the nuclear localization sequence may comprise amino acids with positively charged groups. For example, the nuclear localization sequence may comprise an amino acid sequence of a nuclear localization sequence known in the field. Alternatively, it may have one, two, three, four, five, or more changes compared to the above sequence, such as sequences with amino acid substitutions, insertions, or deletions, or any fragments thereof. For example, the nuclear localization sequence may be located at the N-terminal and/or C-terminal of the first functional domain, the N-terminal and/or C-terminal of the second functional domain, and/or the N-terminal and/or C-terminal of the DNA binding domain.

In some embodiments, the gene expression-regulating molecule may also comprise a detectable portion. For example, the detectable portion may comprise blue fluorescent protein and/or green fluorescent protein. For example, the detectable portion is linked to the first functional domain, the second functional domain, the DNA binding domain, and/or the nuclear localization sequence via a self-cleaving peptide. For example, the self-cleaving peptide may comprise a 2A peptide.

In the present application, the first functional domain, the second functional domain, the DNA binding domain, and other elements contained in the gene-regulating molecules mentioned herein may be linked indirectly, for example, via a linker sequence of a certain length. For example, the linker may comprise about 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 50, 60, 70, 80, 90, 100, or more amino acids. For example, the first functional domain and the second functional domain is linked via a linker of about 80 or more amino acids in length. For example, the first functional domain and the second functional domain is linked via a linker of about 92 or more amino acids in length. For example, the first functional domain and the second functional domain is linked through an XTEN linker. For example, the linker comprises an amino acid sequence of the XTEN linker. For example, the linker comprises an amino acid sequence of the XTEN linker. For example, the linker comprises an amino acid sequence of an XTEN linker of 16 amino acids in length, 80 amino acids in length, or longer. Alternatively, it may have one, two, three, four, five, or more changes compared to the above sequence, such as sequences with amino acid substitutions, insertions, or deletions, or any fragments thereof.

### Nucleic acid-binding molecules

The present application also provides a nucleic acid-binding molecule, wherein the nucleic acid-binding molecule may comprise a sequence of any one of SEQ ID NOs: 1-50. For example, the nucleic acid-binding molecule and/or the gene expression-regulating molecule of the present application may be delivered to the subject by local injection, systemic infusion, or a combination thereof. In all aspects, the nucleic acid-binding molecule may comprise a sequence with at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the sequences of SEQ ID NOs: 1-50.

In some embodiments, the combination of the nucleic acid-binding molecule and the gene expression-regulating molecule of the present application may have the capability to regulate the expression level of the LPA gene. For example, the nucleic acid-binding molecule and/or the gene expression-regulating molecule of the present application may bind to the core promoter, proximal promoter, distal enhancer, silencer, insulator element, boundary element, and/or locus control region of the LPA gene. For example, the nucleic acid-binding molecule and/or the gene expression-regulating molecule of the present application may bind to a DNA region within about 500 bp upstream and/or downstream of the transcription start site of the LPA gene, or a fragment thereof. For example, the nucleic acid-binding molecule and/or the gene expression-regulating molecule of the present application may bind to the DNA region where SEQ ID NO: 51 is located, or a fragment thereof. In all aspects, the DNA region that can be bound may comprise a sequence with at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the DNA region where SEQ ID NO: 51 is located.

For example, the nucleic acid-binding molecule and/or the gene expression-regulating molecule of the present application may bind to a DNA region where any one of SEQ ID NOs: 52-101 is located, or a fragment thereof. In all aspects, the DNA region that can be bound may comprise a sequence with at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the DNA region where any one of SEQ ID NOs: 52-101 is located. For example, targeting any region of about 20 bp in the DNA region where any one of SEQ ID NOs: 52-101 is located may have the capability to regulate the expression level of the LPA gene. For example, compared to the expression and/or activity of the target gene in the absence of the substance of the present application, the administration of the substance of the present application may have a negative impact (e.g., reduction) on the activity of the nucleic acid sequence, which may include at least partially, partially, or completely blocking the activation of the nucleic acid sequence (e.g., transcription), or reducing, preventing, or delaying the activation of the nucleic acid sequence. For example, the inhibition of activity may be about 90%, about 80%, about 70%, about 60%, about 50%, about 40%, about 30%, about 20%, about 10%, or lower of the control.

In some embodiments, the guide sequence or spacer length of the nucleic acid-binding molecule may be 15 to 50 nucleotides. In some embodiments, the nucleic acid-binding molecule is a guide RNA (gRNA), and the spacer length of the guide RNA may be at least 15 nucleotides. In some embodiments, the spacer length may be 15 to 17 nucleotides, 17 to 20 nucleotides, 20 to 24 nucleotides, 23 to 25 nucleotides, 24 to 27 nucleotides, 27 to 30 nucleotides, 30 to 35 nucleotides, or greater than 35 nucleotides. In some embodiments, the number of gRNAs administered may be at least 1 type of gRNA, at least 2 types of different gRNAs, at least 3 types of different gRNAs, at least 4 types of different gRNAs, at least 5 types of different gRNAs. In some embodiments, the length of the target binding region may be between about 19 and about 21 nucleotides. In one embodiment, the length of the target binding region may be 15, 16, 17, 18, 19, 20, 21, 22, 23, 24, or 25 nucleotides. In one embodiment, the target binding region may be complementary to the target region in the target gene, for example, completely complementary. In one embodiment, the target binding region may be substantially complementary to the target region in the target gene. In one embodiment, the target binding region may comprise no more than 1, 2, 3, 4, 5, 6, 7, 8, 9, or 10 nucleotides that are not complementary to the target region in the target gene. In some embodiments, the nucleic acid-binding molecule and/or the gene expression-regulating molecule of the present application may be formulated in liposomes or lipid nanoparticles. In some embodiments, the nucleic acid-binding molecule and/or the gene expression-regulating molecule of the present application may be formulated in viral vectors. For example, the guide RNA may include an RNA-based molecule with one or more chemical modifications (e.g., connecting two ribonucleotides via a chemical linkage or replacing one or more ribonucleotides with one or more deoxyribonucleotides). For example, a target binding region derived from humans may be targeted.

Without wishing to be bound by any particular theory, the examples provided below are merely for illustrative purposes of the products, preparation methods, and uses of the present application, and are not intended to limit the scope of the present invention.

### EXAMPLES

### Example 1

### Epigenetic Regulation Efficiency of the Gene Regulation Molecule of the Present Application in a Reporter Cell Line

(1) Experimental Method: In this example, a sequence between 500 bp before and 500 bp after the transcription start site of the human LPA gene (a total of 1000 bp) was inserted in front of a green fluorescent protein in an expression vector to construct a reporter plasmid (Figure 1A). The reporter plasmid was then stably integrated into HEK293T cells to obtain a reporter system cell line. Meanwhile, a guide RNA (gRNA) plasmid inserted with red fluorescent protein mCherry+ was constructed, and multiple guide sequences were designed on this 1000 bp sequence to guide different epigenetic editing tools (editing tool plasmids with blue fluorescent protein) to the target sites corresponding to these gRNAs. The guidance process conducted by cell transfection of co-transfecting the editing tool plasmid with blue fluorescence and the gRNA plasmid with red fluorescence into the reporter cell line.

### (2) Experimental Materials and Design

The DNA sequence of the reporter system used in this example (standard font: promoter region, bold: LPA gene regulatory region, italic: green fluorescent protein (EGFP) sequence) is as follows:

ATAGTAATCAATTACGGGGTCATTAGTTCATAGCCCATATATGGAGTTCCGCGTTACATAACTTA
CGGTAAATGGCCCGCCTGGCTGACCGCCCAACGACCCCCGCCCATTGACGTCAATAATGACGTA
TGTTCCCATAGTAACGCCAATAGGGACTTTCCATTGACGTCAATGGGTGGAGTATTTACGGTAAA
CTGCCCACTTGGCAGTACATCAAGTGTATCATATGCCAAGTCCGCCCCCTATTGACGTCAATGAC
GGTAAATGGCCCGCCTGGCATTATGCCCAGTACATGACCTTACGGGACTTTCCTACTTGGCAGTA
CATCTACGTATTAGTCATCGCTATTACCATGGTGATGCGGTTTTGGCAGTACACCAATGGGCGTG
GATAGCGGTTTGACTCACGGGGATTTCCAAGTCTCCACCCCATTGACGTCAATGGGAGTTTGTTT
TGGCACCAAAATCAACGGGACTTTCCAAAATGTCGTAATAACCCCGCCCCGTTGACGCAAATGG
GCGGTAGGCGTGTACGGTGGGAGGTCTATATAAGCAGAGGTCGTTTAGTGAACCGTCAGATCAC
GCGTGCTAGCACTAAGACCTGAAAGGGTTGTGTCTATCAGCTGCACCCCTGGGTAGCGACA
CAACCTCGGGAAGGCCTCAGCCCCCTCCTCGTACAGCACTGCCTGTTGGAAAGCTTGAGG
GAGGCTATGGATGTGCAGCACTTGGCAGAGGGTCTGGTCATGGAAGTTACCAGCAAATAT
GAGCTACTTTTATGATTTTATTTTATCCAAAAGAAAGAGAATGAAAGAAGAGGGGAGGAAA
CAAGACTAATCAGGAAAGATGAAGGTCTAGGGGTGAGGGAAGGAGTAAGGAGACATAAAG
GCAATGTGGAGCAGCTGAGGGGGGAAATGGCTTTCACCACTTCCCAGCATCTATTGACATT
GCACTCTCAAATATTTTATAAGACTCTATATTCAAGGTAATGTTTGAACCCTGCTGAGCCAG
TGGCATGGGTCTCTGAGAGAATCATTAACTTAATTTGACTATCTGGTTTGTGGGTGCGTTT
ACTCTCATGTAAGTCAACAATGTCCTGGGATTGGGACACACTTTCTGGGCACTGCTGGCCA
GTCCCAAAATGGAACATAAGGAAGTGGTTCTTCTACTTCTTTTATTTCTGAAATCAGGTAA
GACATAGTTTTTTTAAATTATAAGAATTATTTTTTCTCCCACAATGTAGTAAAAATACATAT
GCCATGGCTTTATGTGCAATTCATTTAATTTTTGATTCATGAAATTCCCAGTTCAAAATCTT
GTATATGATTGAAAAATTCTTAAAAAAATAAGTTTAATTTCCCCGTGAAGACTGTCACGGT
GCTGGAATGAATGGGCAGAAAAAATAATGGTTGATTTTTCTAATCTAAAAGAGTGTGCCTA
CATGATGGCCAGTCTGGCTGAAAAATAAATAGCCATTGTAGCTAACTATGCAAAGGATGGC
TAAGCTCTTCGCTTGGTTCTCAGTTTCATTAATTTATATCATCTCTGTTCAGGTGCCATGCT
CCCCTCACTAGCAAGTTGAAACAATGAAATAACGGATCCCCCCTCTCCCTCCCACCGCCACCAT
GGTGAGCAAGGGCGAGGAGCTGTTCACCGGGGTGGTGCCCATCCTGGTCGAGCTGGACGGCGACGT
AAACGGCCACAAGTTCAGCGTGTCCGGCGAGGGCGAGGGCGATGCCACCTACGGCAAGCTGACCCTG
AAGTTCATCTGCACCACCGGCAAGCTGCCCGTGCCCTGGCCCACCCTCGTGACCACCCTGACCTACG
GCGTGCAGTGCTTCAGCCGCTACCCCGACCACATGAAGCAGCACGACTTCTTCAAGTCCGCCATGCCC
GAAGGCTACGTCCAGGAGCGCACCATCTTCTTCAAGGACGACGGCAACTACAAGACCCGCGCCGAGG
TGAAGTTCGAGGGCGACACCCTGGTGAACCGCATCGAGCTGAAGGGCATCGACTTCAAGGAGGACGG
CAACATCCTGGGGCACAAGCTGGAGTACAACTACAACAGCCACAACGTCTATATCATGGCCGACAAGC
AGAAGAACGGCATCAAGGTGAACTTCAAGATCCGCCACAACATCGAGGACGGCAGCGTGCAGCTCGC

CGACCACTACCAGCAGAACACCCCCATCGGCGACGGCCCCGTGCTGCTGCCCGACAACCACTACCTG
AGCACCCAGTCCGCCCTGAGCAAAGACCCCAACGAGAAGCGCGATCACATGGTCCTGCTGGAGTTCG
TGACCGCCGCCGGGATCACTCTCGGCATGGACGAGCTGTACAAGTAA (SEQ ID NO: 102).

The DNA sequence of the editing tool EpiRegA (Dnmt3a-Dnmt3l-dCas9-KOX1 KRAB) (italic/underlined italic: Dnmt3a/Dnmt3l, bold: dCas9, bold italic: KRAB, underlined bold italic: blue fluorescent protein, standard font: linker sequence) is as follows:

ATGAACCATGACCAGGAATTTGACCCCCCAAAGGTTTACCCACCTGTGCCAGCTGAGAAGAGGAAGCC
CATCCGCGTGCTGTCTCTCTTTGATGGGATTGCTACAGGGCTCCTGGTGCTGAAGGACCTGGGCATCC
AAGTGGACCGCTACATTGCCTCCGAGGTGTGTGAGGACTCCATCACGGTGGGCATGGTGCGGCACCA
GGGAAAGATCATGTACGTCGGGGACGTCCGCAGCGTCACACAGAAGCATATCCAGGAGTGGGGCCCA
TTCGACCTGGTGATTGGAGGCAGTCCCTGCAATGACCTCTCCATTGTCAACCCTGCCCGCAAGGGACT
TTATGAGGGTACTGGCCGCCTCTTCTTTGAGTTCTACCGCCTCCTGCATGATGCGCGGCCCAAGGAGG
GAGATGATCGCCCCTTCTTCTGGCTCTTTGAGAATGTGGTGGCCATGGGCGTTAGTGACAAGAGGGAC
ATCTCGCGATTTCTTGAGTCTAACCCCGTGATGATTGACGCCAAAGAAGTGTCTGCTGCACACAGGGCC
CGTTACTTCTGGGGTAACCTTCCTGGCATGAACAGGCCTTTGGCATCCACTGTGAATGATAAGCTGGAG
CTGCAAGAGTGTCTGGAGCACGGCAGAATAGCCAAGTTCAGCAAAGTGAGGACCATTACCACCAGGTC
AAACTCTATAAAGCAGGGCAAAGACCAGCATTTCCCCGTCTTCATGAACGAGAAGGAGGACATCCTGTG
GTGCACTGAAATGGAAAGGGTGTTTGGCTTCCCCGTCCACTACACAGACGTCTCCAACATGAGCCGCT
TGGCGAGGCAGAGACTGCTGGGCCGATCGTGGAGCGTGCCGGTCATCCGCCACCTCTTCGCTCCGCT
GAAGGAATATTTTGCTTGTGTGTCTAGCGGCAATAGTAACGCTAACAGCCGCGGGCCGAGCTTCAG
CAGCGGCCTGGTGCCGTTAAGCTTGCGCGGCAGCCATATGGGCCCTATGGAGATATACAAGACAGT
GTCTGCATGGAAGAGACAGCCAGTGCGGGTACTGAGCCTCTTCAGAAACATCGACAAGGTACTAAAGA
GTTTGGGCTTCTTGGAAAGCGGTTCTGGTTCTGGGGGAGGAACGCTGAAGTACGTGGAAGATGTCACA
AATGTCGTGAGGAGAGACGTGGAGAAATGGGGCCCCTTTGACCTGGTGTACGGCTCGACGCAGCCCC
TAGGCAGCTCTTGTGATCGCTGTCCCGGCTGGTACATGTTCCAGTTCCACCGGATCCTGCAGTATGCG
CTGCCTCGCCAGGAGAGTCAGCGGCCCTTCTTCTGGATATTCATGGACAATCTGCTGCTGACTGAGGA
TGACCAAGAGACAACTACCCGCTTCCTTCAGACAGAGGCTGTGACCCTCCAGGATGTCCGTGGCAGAG
ACTACCAGAATGCTATGCGGGTGTGGAGCAACATTCCAGGGCTGAAGAGCAAGCATGCGCCCCTGACC
CCAAAGGAAGAAGAGTATCTGCAAGCCCAAGTCAGAAGCAGGAGCAAGCTGGACGCCCCGAAAGTTGA
CCTCCTGGTGAAGAACTGCCTTCTCCCGCTGAGAGAGTACTTCAAGTATTTTTCTCAAAACTCACTTCCT
CTTGGAGGGCCGAGCTCTGGCGCACCCCCACCAAGTGGAGGGTCTCCTGCCGGGTCCCCAACAT
CTACTGAAGAAGGCACCAGCGAATCCGCAACGCCCGAGTCAGGCCCTGGTACCTCCACAGAACC
ATCTGAAGGTAGTGCGCCTGGTTCCCCAGCTGGAAGCCCTACTTCCACCGAAGAAGGCACGTCA
ACCGAACCAAGTGAAGGATCTGCCCCTGGGACCAGCACTGAACCATCTGAGCCAAAAAAGAAG
AGAAAGGTAATGGACAAGAAGTACAGCATCGGCCTGGCCATCGGCACCAACTCTGTGGGCT
GGGCCGTGATCACCGACGAGTACAAGGTGCCCAGCAAGAAATTCAAGGTGCTGGGCAACA
CCGACCGGCACAGCATCAAGAAGAACCTGATCGGCGCCCTGCTGTTCGACAGCGGAGAAA
CAGCCGAGGCCACCCGGCTGAAGAGAACCGCCAGAAGAAGATACACCAGACGGAAGAACC
GGATCTGCTATCTGCAAGAGATCTTCAGCAACGAGATGGCCAAGGTGGACGACAGCTTCTT
CCACAGACTGGAAGAGTCCTTCCTGGTGGAAGAGGATAAGAAGCACGAGCGGCACCCCAT
CTTCGGCAACATCGTGGACGAGGTGGCCTACCACGAGAAGTACCCCACCATCTACCACCTG
AGAAAGAAACTGGTGGACAGCACCGACAAGGCCGACCTGCGGCTGATCTATCTGGCCCTG
GCCCACATGATCAAGTTCCGGGGCCACTTCCTGATCGAGGGCGACCTGAACCCCGACAAC
AGCGACGTGGACAAGCTGTTCATCCAGCTGGTGCAGACCTACAACCAGCTGTTCGAGGAA
AACCCCATCAACGCCAGCGGCGTGGACGCCAAGGCCATCCTGTCTGCCAGACTGAGCAAG
AGCAGACGGCTGGAAAATCTGATCGCCCAGCTGCCCGGCGAGAAGAAGAATGGCCTGTTC
GGCAACCTGATTGCCCTGAGCCTGGGCCTGACCCCCAACTTCAAGAGCAACTTCGACCTGG
CCGAGGATGCCAAACTGCAGCTGAGCAAGGACACCTACGACGACGACCTGGACAACCTGC
TGGCCCAGATCGGCGACCAGTACGCCGACCTGTTTCTGGCCGCCAAGAACCTGTCCGACG
CCATCCTGCTGAGCGACATCCTGAGAGTGAACACCGAGATCACCAAGGCCCCCCTGAGCG
CCTCTATGATCAAGAGATACGACGAGCACCACCAGGACCTGACCCTGCTGAAAGCTCTCGT
GCGGCAGCAGCTGCCTGAGAAGTACAAAGAGATTTTCTTCGACCAGAGCAAGAACGGCTA
CGCCGGCTACATCGATGGCGGAGCCAGCCAGGAAGAGTTCTACAAGTTCATCAAGCCCAT
CCTGGAAAAGATGGACGGCACCGAGGAACTGCTCGTGAAGCTGAACAGAGAGGACCTGCT
GCGGAAGCAGCGGACCTTCGACAACGGCAGCATCCCCCACCAGATCCACCTGGGAGAGCT
GCACGCCATTCTGCGGCGGCAGGAAGATTTTTACCCATTCCTGAAGGACAACCGGGAAAA
GATCGAGAAGATCCTGACCTTCCGCATCCCCTACTACGTGGGCCCTCTGGCCAGGGGAAA
CAGCAGATTCGCCTGGATGACCAGAAAGAGCGAGGAAACCATCACCCCCTGGAACTTCGA
GGAAGTGGTGGACAAGGGCGCCAGCGCCCAGAGCTTCATCGAGCGGATGACCAACTTCGA
TAAGAACCTGCCCAACGAGAAGGTGCTGCCCAAGCACAGCCTGCTGTACGAGTACTTCACC

GTGTACAACGAGCTGACCAAAGTGAAATACGTGACCGAGGGAATGAGAAAGCCCGCCTTC
CTGAGCGGCGAGCAGAAAAAAGCCATCGTGGACCTGCTGTTCAAGACCAACCGGAAAGTG
ACCGTGAAGCAGCTGAAAGAGGACTACTTCAAGAAAATCGAGTGCTTCGACTCCGTGGAA
ATCTCCGGCGTGGAAGATCGGTTCAACGCCTCCCTGGGCACATACCACGATCTGCTGAAAA
TTATCAAGGACAAGGACTTCCTGGACAATGAGGAAAACGAGGACATTCTGGAAGATATCGT
GCTGACCCTGACACTGTTTGAGGACAGAGAGATGATCGAGGAACGGCTGAAAACCTATGC
CCACCTGTTCGACGACAAAGTGATGAAGCAGCTGAAGCGGCGGAGATACACCGGCTGGGG
CAGGCTGAGCCGGAAGCTGATCAACGGCATCCGGGACAAGCAGTCCGGCAAGACAATCCT
GGATTTCCTGAAGTCCGACGGCTTCGCCAACAGAAACTTCATGCAGCTGATCCACGACGAC
AGCCTGACCTTTAAAGAGGACATCCAGAAAGCCCAGGTGTCCGGCCAGGGCGATAGCCTG
CACGAGCACATTGCCAATCTGGCCGGCAGCCCCGCCATTAAGAAGGGCATCCTGCAGACA
GTGAAGGTGGTGGACGAGCTCGTGAAAGTGATGGGCCGGCACAAGCCCGAGAACATCGTG
ATCGAAATGGCCAGAGAGAACCAGACCACCCAGAAGGGACAGAAGAACAGCCGCGAGAGA
ATGAAGCGGATCGAAGAGGGCATCAAAGAGCTGGGCAGCCAGATCCTGAAAGAACACCCC
GTGGAAAACACCCAGCTGCAGAACGAGAAGCTGTACCTGTACTACCTGCAGAATGGGCGG
GATATGTACGTGGACCAGGAACTGGACATCAACCGGCTGTCCGACTACGATGTGGACGCT
ATCGTGCCTCAGAGCTTTCTGAAGGACGACTCCATCGATAACAAAGTGCTGACTCGGAGCG
ACAAGAACCGGGGCAAGAGCGACAACGTGCCCTCCGAAGAGGTCGTGAAGAAGATGAAGA
ACTACTGGCGCCAGCTGCTGAATGCCAAGCTGATTACCCAGAGGAAGTTCGACAATCTGAC
CAAGGCCGAGAGAGGCGGCCTGAGCGAACTGGATAAGGCCGGCTTCATCAAGAGACAGCT
GGTGGAAACCCGGCAGATCACAAAGCACGTGGCACAGATCCTGGACTCCCGGATGAACAC
TAAGTACGACGAGAACGACAAACTGATCCGGGAAGTGAAAGTGATCACCCTGAAGTCCAA
GCTGGTGTCCGATTTCCGGAAGGATTTCCAGTTTTACAAAGTGCGCGAGATCAACAACTAC
CACCACGCCCACGACGCCTACCTGAACGCCGTCGTGGGAACCGCCCTGATCAAAAAGTAC
CCTAAGCTGGAAAGCGAGTTCGTGTACGGCGACTACAAGGTGTACGACGTGCGGAAGATG
ATCGCCAAGAGCGAGCAGGAAATCGGCAAGGCTACCGCCAAGTACTTCTTCTACAGCAAC
ATCATGAACTTTTTCAAGACCGAGATTACCCTGGCCAACGGCGAGATCCGGAAGCGGCCTC
TGATCGAGACAAACGGCGAAACAGGCGAGATCGTGTGGGATAAGGGCCGGGACTTTGCCA
CCGTGCGGAAAGTGCTGTCTATGCCCCAAGTGAATATCGTGAAAAAGACCGAGGTGCAGA
CAGGCGGCTTCAGCAAAGAGTCTATCCTGCCCAAGAGGAACAGCGACAAGCTGATCGCCA
GAAAGAAGGACTGGGACCCTAAGAAGTACGGCGGCTTCGACAGCCCCACCGTGGCCTATT
CTGTGCTGGTGGTGGCCAAAGTGGAAAAGGGCAAGTCCAAGAAACTGAAGAGTGTGAAAG
AGCTGCTGGGGATCACCATCATGGAAAGAAGCAGCTTCGAGAAGAATCCCATCGACTTTCT
GGAAGCCAAGGGCTACAAAGAAGTGAAAAAGGACCTGATCATCAAGCTGCCTAAGTACTC
CCTGTTCGAGCTGGAAAACGGCCGGAAGAGAATGCTGGCCTCTGCCGGCGAACTGCAGAA
GGGAAACGAACTGGCCCTGCCCTCCAAATATGTGAACTTCCTGTACCTGGCCAGCCACTAT
GAGAAGCTGAAGGGCTCCCCCGAGGATAATGAGCAGAAACAGCTGTTTGTGGAACAGCAC
AAACACTACCTGGACGAGATCATCGAGCAGATCAGCGAGTTCTCCAAGAGAGTGATCCTG
GCCGACGCTAATCTGGACAAGGTGCTGAGCGCCTACAACAAGCACAGAGACAAGCCTATC
AGAGAGCAGGCCGAGAATATCATCCACCTGTTTACCCTGACCAATCTGGGAGCCCCTGCCG
CCTTCAAGTACTTTGACACCACCATCGACCGGAAGAGGTACACCAGCACCAAAGAGGTGCT
GGACGCCACCCTGATCCACCAGAGCATCACCGGCCTGTACGAGACACGGATCGACCTGTC
TCAGCTGGGAGGCGACGCCTATCCCTATGACGTGCCCGATTATGCCAGCCTGGGCAGCGGCTC
CCCCAAGAAAAAACGCAAGGTGGAAGATCCTAAGAAAAAGCGGAAAGTGGACGGCAGCGGCA
GCGAGACCCCCGGTACCAGCGAGAGCGCCACCCCCGAGAGCCGGACACTGGTGACCTTCAAGGA
TGTATTTGTGGACTTCACCAGGGAGGAGTGGAAGCTGCTGGACACTGCTCAGCAGATCGTGTACA
GAAATGTGATGCTGGAGAACTATAAGAACCTGGTTTCCTTGGGTTATCAGCTTACTAAGCCAGATG
TGATCCTCCGGTTGGAGAAGGGAGAAGAGCCCGTCGACCTCGAGGCTACTAACTTCAGCCTGCTG
AAGCAGGCTGGAGACGTGGAGGAGAACCCTGGACCTATGCATATGAGCGAGCTGATTAAGGAGA
ACATGCACATGAAGCTGTACATGGAGGGCACCGTGGACAACCATCACTTCAAGTGCACATCCGAG
GGCGAAGGCAAGCCCTACGAGGGCACCCAGACCATGAGAATCAAGGTGGTCGAGGGCGGCCCTCT
CCCCTTCGCCTTCGACATCCTGGCTACTAGCTTCCTCTACGGCAGCAAGACCTTCATCAACCACAC
CCAGGGCATCCCCGACTTCTTCAAGCAGTCCTTCCCTGAGGGCTTCACATGGGAGAGAGTCACCAC
ATACGAAGACGGGGGCGTGCTGACCGCTACCCAGGACACCAGCCTCCAGGACGGCTGCCTCATCT
ACAACGTCAAGATCAGAGGGGTGAACTTCACATCCAACGGCCCTGTGATGCAGAAGAAAACACTC
GGCTGGGAGGCCTTCACCGAGACGCTGTACCCCGCTGACGGCGGCCTGGAAGGCAGAAACGACAT
GGCCCTGAAGCTCGTGGGCGGGAGCCATCTGATCGCAAACATCAAGACCACATATAGATCCAAGA
AACCCGCTAAGAACCTCAAGATGCCTGGCGTCTACTATGTGGACTACAGACTGGAAAGAATCAAGG
AGGCCAACAACGAGACCTACGTCGAGCAGCACGAGGTGGCAGTGGCCAGATACTGCGACCTCCCT
AGCAAACTGGGGCACAAGCTTAATTAA (SEQ ID NO:103).

The amino acid sequence of the editing tool EpiRegA (Dnmt3a-Dnmt3l-dCas9-KOX1 KRAB) (italic/underlined italic: Dnmt3a/Dnmt3l, bold: dCas9, bold italic: KRAB, underlined bold italic: blue fluorescent protein, standard font: linker sequence, *: any amino acid) is as follows:

MNHDQEFDPPKVYPPVPAEKRKPIRVLSLFDGIATGLLVLKDLGIQVDRYIASEVCEDSITVGMVRHQGKIM
YVGDVRSVTQKHIQEWGPFDLVIGGSPCNDLSIVNPARKGLYEGTGRLFFEFYRLLHDARPKEGDDRPFF
WLFENVVAMGVSDKRDISRFLESNPVMIDAKEVSAAHRARYFWGNLPGMNRPLASTVNDKLELQECLEHG
RIAKFSKVRTITTRSNSIKQGKDQHFPVFMNEKEDILWCTEMERVFGFPVHYTDVSNMSRLARQRLLGRSW
SVPVIRHLFAPLKEYFACVSSGNSNANSRGPSFSSGLVPLSLRGSHMGPMEIYKTVSAWKRQPVRVLSLFR
NIDKVLKSLGFLESGSGSGGGTLKYVEDVTNVVRRDVEKWGPFDLVYGSTQPLGSSCDRCPGWYMFQFHR
ILQYALPRQESQRPFFWIFMDNLLLTEDDQETTTRFLQTEAVTLQDVRGRDYQNAMRVWSNIPGLKSKHAP
LTPKEEEYLQAQVRSRSKLDAPKVDLLVKNCLLPLREYFKYFSQNSLPLGGPSSGAPPPSGGSPAGSPTSTE
EGTSESATPESGPGTSTEPSEGSAPGSPAGSPTSTEEGTSTEPSEGSAPGTSTEPSEPKKKRKVMDKKY
SIGLAIGTNSVGWAVITDEYKVPSKKFKVLGNTDRHSIKKNLIGALLFDSGETAEATRLKRTAR
RRYTRRKNRICYLQEIFSNEMAKVDDSFFHRLEESFLVEEDKKHERHPIFGNIVDEVAYHEKYP
TIYHLRKKLVDSTDKADLRLIYLALAHMIKFRGHFLIEGDLNPDNSDVDKLFIQLVQTYNQLFE
ENPINASGVDAKAILSARLSKSRRLENLIAQLPGEKKNGLFGNLIALSLGLTPNFKSNFDLAEDA
KLQLSKDTYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMDGTEELLV
KLNREDLLRKQRTFDNGSIPHQIHLGELHAILRRQEDFYPFLKDNREKIEKILTFRIPYYVGPLA
RGNSRFAWMTRKSEETITPWNFEEVVDKGASAQSFIERMTNFDKNLPNEKVLPKHSLLYEYFT
VYNELTKVKYVTEGMRKPAFLSGEQKKAIVDLLFKTNRKVTVKQLKEDYFKKIECFDSVEISG
VEDRFNASLGTYHDLLKIIKDKDFLDNEENEDILEDIVLTLTLFEDREMIEERLKTYAHLFDDKV
MKQLKRRRYTGWGRLSRKLINGIRDKQSGKTILDFLKSDGFANRNFMQLIHDDSLTFKEDIQK
AQVSGQGDSLHEHIANLAGSPAIKKGILQTVKVVDELVKVMGRHKPENIVIEMARENQTTQKG
QKNSRERMKRIEEGIKELGSQILKEHPVENTQLQNEKLYLYYLQNGRDMYVDQELDINRLSDY
DVDAIVPQSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVITLKSKLV
SDFRKDFQFYKVREINNYHHAHDAYLNAVVGTALIKKYPKLESEFVYGDYKVYDVRKMIAKSE
QEIGKATAKYFFYSNIMNFFKTEITLANGEIRKRPLIETNGETGEIVWDKGRDFATVRKVLSMP
QVNIVKKTEVQTGGFSKESILPKRNSDKLIARKKDWDPKKYGGFDSPTVAYSVLVVAKVEKGK
SKKLKSVKELLGITIMERSSFEKNPIDFLEAKGYKEVKKDLIIKLPKYSLFELENGRKRMLASAG
ELQKGNELALPSKYVNFLYLASHYEKLKGSPEDNEQKQLFVEQHKHYLDEIIEQISEFSKRVILA
DANLDKVLSAYNKHRDKPIREQAENIIHLFTLTNLGAPAAFKYFDTTIDRKRYTSTKEVLDATLI
HQSITGLYETRIDLSQLGGDAYPYDVPDYASLGSGSPKKKRKVEDPKKKRKVDGSGSETPGTSESA
TPESRTLVTFKDVFVDFTREEWKLLDTAQQIVYRNVMLENYKNLVSLGYQLTKPDVILRLEKGEEPV
DLEATNFSLLKQAGDVEENPGPMHMSELIKENMHMKLYMEGTVDNHHFKCTSEGEGKPYEGTQT
MRIKVVEGGPLPFAFDILATSFLYGSKTFINHTQGIPDFFKQSFPEGFTWERVTTYEDGGVLTATQDT
SLQDGCLIYNVKIRGVNFTSNGPVMQKKTLGWEAFTETLYPADGGLEGRNDMALKLVGGSHLIANIK
TTYRSKKPAKNLKMPGVYYVDYRLERIKEANNETYVEQHEVAVARYCDLPSKLGHKLN*(SEQ ID
NO:104).

The DNA sequence of the editing tool EpiRegB (Dnmt3a-Dnmt3l-ZIM3 KRAB-dCas9) (italic/underlined italic: Dnmt3a/Dnmt3l, bold: dCas9, bold italic: KRAB, underlined bold italic: blue fluorescent protein, standard font: linker sequence) is as follows:

ATGAACCATGACCAGGAATTTGACCCCCCAAAGGTTTACCCACCTGTGCCAGCTGAGAAGAGGAAGCC
CATCCGCGTGCTGTCTCTCTTTGATGGGATTGCTACAGGGCTCCTGGTGCTGAAGGACCTGGGCATCC
AAGTGGACCGCTACATTGCCTCCGAGGTGTGTGAGGACTCCATCACGGTGGGCATGGTGCGGCACCA
GGGAAAGATCATGTACGTCGGGGACGTCCGCAGCGTCACACAGAAGCATATCCAGGAGTGGGGCCCA
TTCGACCTGGTGATTGGAGGCAGTCCCTGCAATGACCTCTCCATTGTCAACCCTGCCCGCAAGGGACT
TTATGAGGGTACTGGCCGCCTCTTCTTTGAGTTCTACCGCCTCCTGCATGATGCGCGGCCCAAGGAGG
GAGATGATCGCCCCTTCTTCTGGCTCTTTGAGAATGTGGTGGCCATGGGCGTTAGTGACAAGAGGGAC
ATCTCGCGATTTCTTGAGTCTAACCCCGTGATGATTGACGCCAAAGAAGTGTCTGCTGCACACAGGGCC
CGTTACTTCTGGGGTAACCTTCCTGGCATGAACAGGCCTTTGGCATCCACTGTGAATGATAAGCTGGAG
CTGCAAGAGTGTCTGGAGCACGGCAGAATAGCCAAGTTCAGCAAAGTGAGGACCATTACCACCAGGTC
AAACTCTATAAAGCAGGGCAAAGACCAGCATTTCCCCGTCTTCATGAACGAGAAGGAGGACATCCTGTG
GTGCACTGAAATGGAAAGGGTGTTTGGCTTCCCCGTCCACTACACAGACGTCTCCAACATGAGCCGCT
TGGCGAGGCAGAGACTGCTGGGCCGATCGTGGAGCGTGCCGGTCATCCGCCACCTCTTCGCTCCGCT
GAAGGAATATTTTGCTTGTGTGTCTAGCGGCAATAGTAACGCTAACAGCCGCGGGCCGAGCTTCAG
CAGCGGCCTGGTGCCGTTAAGCTTGCGCGGCAGCCATATGGGCCCTATGGAGATATACAAGACAGT
GTCTGCATGGAAGAGACAGCCAGTGCGGGTACTGAGCCTCTTCAGAAACATCGACAAGGTACTAAAGA
GTTTGGGCTTCTTGGAAAGCGGTTCTGGTTCTGGGGGAGGAACGCTGAAGTACGTGGAAGATGTCACA

AATGTCGTGAGGAGAGACGTGGAGAAATGGGGCCCCTTTGACCTGGTGTACGGCTCGACGCAGCCCC
TAGGCAGCTCTTGTGATCGCTGTCCCGGCTGGTACATGTTCCAGTTCCACCGGATCCTGCAGTATGCG
CTGCCTCGCCAGGAGAGTCAGCGGCCCTTCTTCTGGATATTCATGGACAATCTGCTGCTGACTGAGGA
TGACCAAGAGACAACTACCCGCTTCCTTCAGACAGAGGCTGTGACCCTCCAGGATGTCCGTGGCAGAG
ACTACCAGAATGCTATGCGGGTGTGGAGCAACATTCCAGGGCTGAAGAGCAAGCATGCGCCCCTGACC
CCAAAGGAAGAAGAGTATCTGCAAGCCCAAGTCAGAAGCAGGAGCAAGCTGGACGCCCCGAAAGTTGA
CCTCCTGGTGAAGAACTGCCTTCTCCCGCTGAGAGAGTACTTCAAGTATTTTTCTCAAAACTCACTTCCT
CTTGACGGCAGCGGCAGCGAGACCCCCGGTACCAGCGAGAGCGCCACCCCCGAGAGCATGAACA
ATTCCCAGGGAAGAGTGACCTTCGAGGATGTCACTGTGAACTTCACCCAGGGGGAGTGGCAGCGG
CTGAATCCCGAACAGAGAAACTTGTACAGGGATGTGATGCTGGAGAATTACAGCAACCTTGTCTCT
GTGGGACAAGGGGAAACCACCAAACCCGATGTGATCTTGAGGTTGGAACAAGGAAAGGAGCCATG
GTTGGAGGAAGAGGAAGTGCTGGGAAGTGGCCGTGCAGAAAAAAATGGGGACATTGGAGGGCAG
ATTTGGAAGCCAAAGGATGTGAAAGAGAGTCTCGGAGGGCCGAGCTCTGGCGCACCCCCACCAA
GTGGAGGGTCTCCTGCCGGGTCCCCAACATCTACTGAAGAAGGCACCAGCGAATCCGCAACGCC
CGAGTCAGGCCCTGGTACCTCCACAGAACCATCTGAAGGTAGTGCGCCTGGTTCCCCAGCTGGA
AGCCCTACTTCCACCGAAGAAGGCACGTCAACCGAACCAAGTGAAGGATCTGCCCCTGGGACCA
GCACTGAACCATCTGAGCCAAAAAAGAAGAGAAAGGTAATGGACAAGAAGTACAGCATCGGC
CTGGCCATCGGCACCAACTCTGTGGGCTGGGCCGTGATCACCGACGAGTACAAGGTGCCC
AGCAAGAAATTCAAGGTGCTGGGCAACACCGACCGGCACAGCATCAAGAAGAACCTGATC
GGCGCCCTGCTGTTCGACAGCGGAGAAACAGCCGAGGCCACCCGGCTGAAGAGAACCGCC
AGAAGAAGATACACCAGACGGAAGAACCGGATCTGCTATCTGCAAGAGATCTTCAGCAAC
GAGATGGCCAAGGTGGACGACAGCTTCTTCCACAGACTGGAAGAGTCCTTCCTGGTGGAA
GAGGATAAGAAGCACGAGCGGCACCCCATCTTCGGCAACATCGTGGACGAGGTGGCCTAC
CACGAGAAGTACCCCACCATCTACCACCTGAGAAAGAAACTGGTGGACAGCACCGACAAG
GCCGACCTGCGGCTGATCTATCTGGCCCTGGCCCACATGATCAAGTTCCGGGGCCACTTCC
TGATCGAGGGCGACCTGAACCCCGACAACAGCGACGTGGACAAGCTGTTCATCCAGCTGG
TGCAGACCTACAACCAGCTGTTCGAGGAAAACCCCATCAACGCCAGCGGCGTGGACGCCA
AGGCCATCCTGTCTGCCAGACTGAGCAAGAGCAGACGGCTGGAAAATCTGATCGCCCAGC
TGCCCGGCGAGAAGAAGAATGGCCTGTTCGGCAACCTGATTGCCCTGAGCCTGGGCCTGA
CCCCCAACTTCAAGAGCAACTTCGACCTGGCCGAGGATGCCAAACTGCAGCTGAGCAAGG
ACACCTACGACGACGACCTGGACAACCTGCTGGCCCAGATCGGCGACCAGTACGCCGACC
TGTTTCTGGCCGCCAAGAACCTGTCCGACGCCATCCTGCTGAGCGACATCCTGAGAGTGAA
CACCGAGATCACCAAGGCCCCCCTGAGCGCCTCTATGATCAAGAGATACGACGAGCACCA
CCAGGACCTGACCCTGCTGAAAGCTCTCGTGCGGCAGCAGCTGCCTGAGAAGTACAAAGA
GATTTTCTTCGACCAGAGCAAGAACGGCTACGCCGGCTACATCGATGGCGGAGCCAGCCA
GGAAGAGTTCTACAAGTTCATCAAGCCCATCCTGGAAAAGATGGACGGCACCGAGGAACT
GCTCGTGAAGCTGAACAGAGAGGACCTGCTGCGGAAGCAGCGGACCTTCGACAACGGCAG
CATCCCCCACCAGATCCACCTGGGAGAGCTGCACGCCATTCTGCGGCGGCAGGAAGATTT
TTACCCATTCCTGAAGGACAACCGGGAAAAGATCGAGAAGATCCTGACCTTCCGCATCCCC
TACTACGTGGGCCCTCTGGCCAGGGGAAACAGCAGATTCGCCTGGATGACCAGAAAGAGC
GAGGAAACCATCACCCCCTGGAACTTCGAGGAAGTGGTGGACAAGGGCGCCAGCGCCCAG
AGCTTCATCGAGCGGATGACCAACTTCGATAAGAACCTGCCCAACGAGAAGGTGCTGCCC
AAGCACAGCCTGCTGTACGAGTACTTCACCGTGTACAACGAGCTGACCAAAGTGAAATACG
TGACCGAGGGAATGAGAAAGCCCGCCTTCCTGAGCGGCGAGCAGAAAAAAGCCATCGTGG
ACCTGCTGTTCAAGACCAACCGGAAAGTGACCGTGAAGCAGCTGAAAGAGGACTACTTCA
AGAAAATCGAGTGCTTCGACTCCGTGGAAATCTCCGGCGTGGAAGATCGGTTCAACGCCT
CCCTGGGCACATACCACGATCTGCTGAAAATTATCAAGGACAAGGACTTCCTGGACAATGA
GGAAAACGAGGACATTCTGGAAGATATCGTGCTGACCCTGACACTGTTTGAGGACAGAGA
GATGATCGAGGAACGGCTGAAAACCTATGCCCACCTGTTCGACGACAAAGTGATGAAGCA
GCTGAAGCGGCGGAGATACACCGGCTGGGGCAGGCTGAGCCGGAAGCTGATCAACGGCAT
CCGGGACAAGCAGTCCGGCAAGACAATCCTGGATTTCCTGAAGTCCGACGGCTTCGCCAA
CAGAAACTTCATGCAGCTGATCCACGACGACAGCCTGACCTTTAAAGAGGACATCCAGAAA
GCCCAGGTGTCCGGCCAGGGCGATAGCCTGCACGAGCACATTGCCAATCTGGCCGGCAGC
CCCGCCATTAAGAAGGGCATCCTGCAGACAGTGAAGGTGGTGGACGAGCTCGTGAAAGTG
ATGGGCCGGCACAAGCCCGAGAACATCGTGATCGAAATGGCCAGAGAGAACCAGACCACC
CAGAAGGGACAGAAGAACAGCCGCGAGAGAATGAAGCGGATCGAAGAGGGCATCAAAGA
GCTGGGCAGCCAGATCCTGAAAGAACACCCCGTGGAAAACACCCAGCTGCAGAACGAGAA
GCTGTACCTGTACTACCTGCAGAATGGGCGGGATATGTACGTGGACCAGGAACTGGACAT
CAACCGGCTGTCCGACTACGATGTGGACGCTATCGTGCCTCAGAGCTTTCTGAAGGACGA
CTCCATCGATAACAAAGTGCTGACTCGGAGCGACAAGAACCGGGGCAAGAGCGACAACGT

GCCCTCCGAAGAGGTCGTGAAGAAGATGAAGAACTACTGGCGCCAGCTGCTGAATGCCAA
GCTGATTACCCAGAGGAAGTTCGACAATCTGACCAAGGCCGAGAGAGGCGGCCTGAGCGA
ACTGGATAAGGCCGGCTTCATCAAGAGACAGCTGGTGGAAACCCGGCAGATCACAAAGCA
CGTGGCACAGATCCTGGACTCCCGGATGAACACTAAGTACGACGAGAACGACAAACTGAT
CCGGGAAGTGAAAGTGATCACCCTGAAGTCCAAGCTGGTGTCCGATTTCCGGAAGGATTT
CCAGTTTTACAAAGTGCGCGAGATCAACAACTACCACCACGCCCACGACGCCTACCTGAAC
GCCGTCGTGGGAACCGCCCTGATCAAAAAGTACCCTAAGCTGGAAAGCGAGTTCGTGTAC
GGCGACTACAAGGTGTACGACGTGCGGAAGATGATCGCCAAGAGCGAGCAGGAAATCGGC
AAGGCTACCGCCAAGTACTTCTTCTACAGCAACATCATGAACTTTTTCAAGACCGAGATTA
CCCTGGCCAACGGCGAGATCCGGAAGCGGCCTCTGATCGAGACAAACGGCGAAACAGGCG
AGATCGTGTGGGATAAGGGCCGGGACTTTGCCACCGTGCGGAAAGTGCTGTCTATGCCCC
AAGTGAATATCGTGAAAAAGACCGAGGTGCAGACAGGCGGCTTCAGCAAAGAGTCTATCC
TGCCCAAGAGGAACAGCGACAAGCTGATCGCCAGAAAGAAGGACTGGGACCCTAAGAAGT
ACGGCGGCTTCGACAGCCCCACCGTGGCCTATTCTGTGCTGGTGGTGGCCAAAGTGGAAA
AGGGCAAGTCCAAGAAACTGAAGAGTGTGAAAGAGCTGCTGGGGATCACCATCATGGAAA
GAAGCAGCTTCGAGAAGAATCCCATCGACTTTCTGGAAGCCAAGGGCTACAAAGAAGTGA
AAAAGGACCTGATCATCAAGCTGCCTAAGTACTCCCTGTTCGAGCTGGAAAACGGCCGGAA
GAGAATGCTGGCCTCTGCCGGCGAACTGCAGAAGGGAAACGAACTGGCCCTGCCCTCCAA
ATATGTGAACTTCCTGTACCTGGCCAGCCACTATGAGAAGCTGAAGGGCTCCCCCGAGGAT
AATGAGCAGAAACAGCTGTTTGTGGAACAGCACAAACACTACCTGGACGAGATCATCGAG
CAGATCAGCGAGTTCTCCAAGAGAGTGATCCTGGCCGACGCTAATCTGGACAAGGTGCTG
AGCGCCTACAACAAGCACAGAGACAAGCCTATCAGAGAGCAGGCCGAGAATATCATCCAC
CTGTTTACCCTGACCAATCTGGGAGCCCCTGCCGCCTTCAAGTACTTTGACACCACCATCG
ACCGGAAGAGGTACACCAGCACCAAAGAGGTGCTGGACGCCACCCTGATCCACCAGAGCA
TCACCGGCCTGTACGAGACACGGATCGACCTGTCTCAGCTGGGAGGCGACGCCTATCCCTA
TGACGTGCCCGATTATGCCAGCCTGGGCAGCGGCTCCCCCAAGAAAAAACGCAAGGTGGAAGAT
CCTAAGAAAAAGCGGAAAGTGGACGGCCTCGAGGCTACTAACTTCAGCCTGCTGAAGCAGGCTG
GAGACGTGGAGGAGAACCCTGGACCTATGCATGTGAGCGAGCTGATTAAGGAGAACATGCACAT
GAAGCTGTACATGGAGGGCACCGTGGACAACCATCACTTCAAGTGCACATCCGAGGGCGAAGGCA
AGCCCTACGAGGGCACCCAGACCATGAGAATCAAGGTGGTCGAGGGCGGCCCTCTCCCCTTCGCC
TTCGACATCCTGGCTACTAGCTTCCTCTACGGCAGCAAGACCTTCATCAACCACACCCAGGGCATC
CCCGACTTCTTCAAGCAGTCCTTCCCTGAGGGCTTCACATGGGAGAGAGTCACCACATACGAAGAC
GGGGGCGTGCTGACCGCTACCCAGGACACCAGCCTCCAGGACGGCTGCCTCATCTACAACGTCAA
GATCAGAGGGGTGAACTTCACATCCAACGGCCCTGTGATGCAGAAGAAAACACTCGGCTGGGAGG
CCTTCACCGAAACGCTGTACCCCGCTGACGGCGGCCTGGAAGGCAGAAACGACATGGCCCTGAAG
CTCGTGGGCGGGAGCCATCTGATCGCAAACATCAAGACCACATATAGATCCAAGAAACCCGCTAA
GAACCTCAAGATGCCTGGCGTCTACTATGTGGACTACAGACTGGAAAGAATCAAGGAGGCCAACA
ACGAGACCTACGTCGAGCAGCACGAGGTGGCAGTGGCCAGATACTGCGACCTCCCTAGCAAACTG
GGGCACAAGCTTAATTAA(SEQ ID NO:105).

The amino acid sequence of the editing tool EpiRegB (Dnmt3a-Dnmt3l-ZIM3 KRAB-dCas9) (italic/underline italic: Dnmt3a/Dnmt3l, bold: dCas9, bold italic: KRAB, underline bold italic: blue fluorescent protein, standard font: linker sequence, *: any amino acid) is as follows:

MNHDQEFDPPKVYPPVPAEKRKPIRVLSLFDGIATGLLVLKDLGIQVDRYIASEVCEDSITVGMVRHQGKIM
YVGDVRSVTQKHIQEWGPFDLVIGGSPCNDLSIVNPARKGLYEGTGRLFFEFYRLLHDARPKEGDDRPFF
WLFENVVAMGVSDKRDISRFLESNPVMIDAKEVSAAHRARYFWGNLPGMNRPLASTVNDKLELQECLEHG
RIAKFSKVRTITTRSNSIKQGKDQHFPVFMNEKEDILWCTEMERVFGFPVHYTDVSNMSRLARQRLLGRSW
SVPVIRHLFAPLKEYFACVSSGNSNANSRGPSFSSGLVPLSLRGSHMGPMEIYKTVSAWKRQPVRVLSLFR
NIDKVLKSLGFLESGSGSGGGTLKYVEDVTNVVRRDVEKWGPFDLVYGSTQPLGSSCDRCPGWYMFQFHR
ILQYALPRQESQRPFFWIFMDNLLLTEDDQETTTRFLQTEAVTLQDVRGRDYQNAMRVWSNIPGLKSKHAP
LTPKEEEYLQAQVRSRSKLDAPKVDLLVKNCLLPLREYFKYFSQNSLPLDGSGSETPGTSESATPESMNNS
QGRVTFEDVTVNFTQGEWQRLNPEQRNLYRDVMLENYSNLVSVGQGETTKPDVILRLEQGKEPWL
EEEEVLGSGRAEKNGDIGGQIWKPKDVKESLGGPSSGAPPPSGGSPAGSPTSTEEGTSESATPESGPGT
STEPSEGSAPGSPAGSPTSTEEGTSTEPSEGSAPGTSTEPSEPKKKRKVMDKKYSIGLAIGTNSVGWA
VITDEYKVPSKKFKVLGNTDRHSIKKNLIGALLFDSGETAEATRLKRTARRRYTRRKNRICYLQ
EIFSNEMAKVDDSFFHRLEESFLVEEDKKHERHPIFGNIVDEVAYHEKYPTIYHLRKKLVDSTD
KADLRLIYLALAHMIKFRGHFLIEGDLNPDNSDVDKLFIQLVQTYNQLFEENPINASGVDAKAIL
SARLSKSRRLENLIAQLPGEKKNGLFGNLIALSLGLTPNFKSNFDLAEDAKLQLSKDTYDDDLD
NLLAQIGDQYADLFLAAKNLSDAILLSDILRVNTEITKAPLSASMIKRYDEHHQDLTLLKALVRQ
QLPEKYKEIFFDQSKNGYAGYIDGGASQEEFYKFIKPILEKMDGTEELLVKLNREDLLRKQRTF
DNGSIPHQIHLGELHAILRRQEDFYPFLKDNREKIEKILTFRIPYYVGPLARGNSRFAWMTRKSE

ETITPWNFEEVVDKGASAQSFIERMTNFDKNLPNEKVLPKHSLLYEYFTVYNELTKVKYVTEG
MRKPAFLSGEQKKAIVDLLFKTNRKVTVKQLKEDYFKKIECFDSVEISGVEDRFNASLGTYHDL
LKIIKDKDFLDNEENEDILEDIVLTLTLFEDREMIEERLKTYAHLFDDKVMKQLKRRRYTGWG
RLSRKLINGIRDKQSGKTILDFLKSDGFANRNFMQLIHDDSLTFKEDIQKAQVSGQGDSLHEHIA
NLAGSPAIKKGILQTVKVVDELVKVMGRHKPENIVIEMARENQTTQKGQKNSRERMKRIEEGI
KELGSQILKEHPVENTQLQNEKLYLYYLQNGRDMYVDQELDINRLSDYDVDAIVPQSFLKDDSI
DNKVLTRSDKNRGKSDNVPSEEVVKKMKNYWRQLLNAKLITQRKFDNLTKAERGGLSELDKA
GFIKRQLVETRQITKHVAQILDSRMNTKYDENDKLIREVKVITLKSKLVSDFRKDFQFYKVREI
NNYHHAHDAYLNAVVGTALIKKYPKLESEFVYGDYKVYDVRKMIAKSEQEIGKATAKYFFYSN
IMNFFKTEITLANGEIRKRPLIETNGETGEIVWDKGRDFATVRKVLSMPQVNIVKKTEVQTGGF
SKESILPKRNSDKLIARKKDWDPKKYGGFDSPTVAYSVLVVAKVEKGKSKKLKSVKELLGITIM
ERSSFEKNPIDFLEAKGYKEVKKDLIIKLPKYSLFELENGRKRMLASAGELQKGNELALPSKYV
NFLYLASHYEKLKGSPEDNEQKQLFVEQHKHYLDEIIEQISEFSKRVILADANLDKVLSAYNKH
RDKPIREQAENIIHLFTLTNLGAPAAFKYFDTTIDRKRYTSTKEVLDATLIHQSITGLYETRIDLS
QLGGDAYPYDVPDYASLGSGSPKKKRKVEDPKKKRKVDGLEATNFSLLKQAGDVEENPGPMHVSE
LIKENMHMKLYMEGTVDNHHFKCTSEGEGKPYEGTQTMRIKVVEGGPLPFAFDILATSFLYGSKTFI
NHTQGIPDFFKQSFPEGFTWERVTTYEDGGVLTATQDTSLQDGCLIYNVKIRGVNFTSNGPVMQKKT
LGWEAFTETLYPADGGLEGRNDMALKLVGGSHLIANIKTTYRSKKPAKNLKMPGVYYVDYRLERIKE
ANNETYVEQHEVAVARYCDLPSKLGHKLN*(SEQ ID NO:106).

The DNA sequence of the editing tool EpiRegC (dCas9-ZIM3 KRAB-DNMT3L-Dnmt3a) (italic/underline italic: Dnmt3a/Dnmt3l, bold: dCas9, bold italic: KRAB, underline bold italic: blue fluorescent protein, standard font: linker sequence) is as follows:

ATGCCAAAAAAGAAGAGAAAGGTAATGGACAAGAAGTACAGCATCGGCCTGGCCATCGGCA
CCAACTCTGTGGGCTGGGCCGTGATCACCGACGAGTACAAGGTGCCCAGCAAGAAATTCA
AGGTGCTGGGCAACACCGACCGGCACAGCATCAAGAAGAACCTGATCGGCGCCCTGCTGT
TCGACAGCGGAGAAACAGCCGAGGCCACCCGGCTGAAGAGAACCGCCAGAAGAAGATACA
CCAGACGGAAGAACCGGATCTGCTATCTGCAAGAGATCTTCAGCAACGAGATGGCCAAGG
TGGACGACAGCTTCTTCCACAGACTGGAAGAGTCCTTCCTGGTGGAAGAGGATAAGAAGC
ACGAGCGGCACCCCATCTTCGGCAACATCGTGGACGAGGTGGCCTACCACGAGAAGTACC
CCACCATCTACCACCTGAGAAAGAAACTGGTGGACAGCACCGACAAGGCCGACCTGCGGC
TGATCTATCTGGCCCTGGCCCACATGATCAAGTTCCGGGGCCACTTCCTGATCGAGGGCGA
CCTGAACCCCGACAACAGCGACGTGGACAAGCTGTTCATCCAGCTGGTGCAGACCTACAA
CCAGCTGTTCGAGGAAAACCCCATCAACGCCAGCGGCGTGGACGCCAAGGCCATCCTGTC
TGCCAGACTGAGCAAGAGCAGACGGCTGGAAAATCTGATCGCCCAGCTGCCCGGCGAGAA
GAAGAATGGCCTGTTCGGCAACCTGATTGCCCTGAGCCTGGGCCTGACCCCCAACTTCAAG
AGCAACTTCGACCTGGCCGAGGATGCCAAACTGCAGCTGAGCAAGGACACCTACGACGAC
GACCTGGACAACCTGCTGGCCCAGATCGGCGACCAGTACGCCGACCTGTTTCTGGCCGCC
AAGAACCTGTCCGACGCCATCCTGCTGAGCGACATCCTGAGAGTGAACACCGAGATCACC
AAGGCCCCCCTGAGCGCCTCTATGATCAAGAGATACGACGAGCACCACCAGGACCTGACC
CTGCTGAAAGCTCTCGTGCGGCAGCAGCTGCCTGAGAAGTACAAAGAGATTTTCTTCGACC
AGAGCAAGAACGGCTACGCCGGCTACATCGATGGCGGAGCCAGCCAGGAAGAGTTCTACA
AGTTCATCAAGCCCATCCTGGAAAAGATGGACGGCACCGAGGAACTGCTCGTGAAGCTGA
ACAGAGAGGACCTGCTGCGGAAGCAGCGGACCTTCGACAACGGCAGCATCCCCCACCAGA
TCCACCTGGGAGAGCTGCACGCCATTCTGCGGCGGCAGGAAGATTTTTACCCATTCCTGAA
GGACAACCGGGAAAAGATCGAGAAGATCCTGACCTTCCGCATCCCCTACTACGTGGGCCC
TCTGGCCAGGGGAAACAGCAGATTCGCCTGGATGACCAGAAAGAGCGAGGAAACCATCAC
CCCCTGGAACTTCGAGGAAGTGGTGGACAAGGGCGCCAGCGCCCAGAGCTTCATCGAGCG
GATGACCAACTTCGATAAGAACCTGCCCAACGAGAAGGTGCTGCCCAAGCACAGCCTGCT
GTACGAGTACTTCACCGTGTACAACGAGCTGACCAAAGTGAAATACGTGACCGAGGGAAT
GAGAAAGCCCGCCTTCCTGAGCGGCGAGCAGAAAAAAGCCATCGTGGACCTGCTGTTCAA
GACCAACCGGAAAGTGACCGTGAAGCAGCTGAAAGAGGACTACTTCAAGAAAATCGAGTG
CTTCGACTCCGTGGAAATCTCCGGCGTGGAAGATCGGTTCAACGCCTCCCTGGGCACATAC
CACGATCTGCTGAAAATTATCAAGGACAAGGACTTCCTGGACAATGAGGAAAACGAGGAC
ATTCTGGAAGATATCGTGCTGACCCTGACACTGTTTGAGGACAGAGAGATGATCGAGGAA
CGGCTGAAAACCTATGCCCACCTGTTCGACGACAAAGTGATGAAGCAGCTGAAGCGGCGG
AGATACACCGGCTGGGGCAGGCTGAGCCGGAAGCTGATCAACGGCATCCGGGACAAGCAG
TCCGGCAAGACAATCCTGGATTTCCTGAAGTCCGACGGCTTCGCCAACAGAAACTTCATGC
AGCTGATCCACGACGACAGCCTGACCTTTAAAGAGGACATCCAGAAAGCCCAGGTGTCCG
GCCAGGGCGATAGCCTGCACGAGCACATTGCCAATCTGGCCGGCAGCCCCGCCATTAAGA
AGGGCATCCTGCAGACAGTGAAGGTGGTGGACGAGCTCGTGAAAGTGATGGGCCGGCACA

AGCCCGAGAACATCGTGATCGAAATGGCCAGAGAGAACCAGACCACCCAGAAGGGACAGA
AGAACAGCCGCGAGAGAATGAAGCGGATCGAAGAGGGCATCAAAGAGCTGGGCAGCCAG
ATCCTGAAAGAACACCCCGTGGAAAACACCCAGCTGCAGAACGAGAAGCTGTACCTGTACT
ACCTGCAGAATGGGCGGGATATGTACGTGGACCAGGAACTGGACATCAACCGGCTGTCCG
ACTACGATGTGGACGCTATCGTGCCTCAGAGCTTTCTGAAGGACGACTCCATCGATAACAA
AGTGCTGACTCGGAGCGACAAGAACCGGGGCAAGAGCGACAACGTGCCCTCCGAAGAGGT
CGTGAAGAAGATGAAGAACTACTGGCGCCAGCTGCTGAATGCCAAGCTGATTACCCAGAG
GAAGTTCGACAATCTGACCAAGGCCGAGAGAGGCGGCCTGAGCGAACTGGATAAGGCCGG
CTTCATCAAGAGACAGCTGGTGGAAACCCGGCAGATCACAAAGCACGTGGCACAGATCCT
GGACTCCCGGATGAACACTAAGTACGACGAGAACGACAAACTGATCCGGGAAGTGAAAGT
GATCACCCTGAAGTCCAAGCTGGTGTCCGATTTCCGGAAGGATTTCCAGTTTTACAAAGTG
CGCGAGATCAACAACTACCACCACGCCCACGACGCCTACCTGAACGCCGTCGTGGGAACC
GCCCTGATCAAAAAGTACCCTAAGCTGGAAAGCGAGTTCGTGTACGGCGACTACAAGGTG
TACGACGTGCGGAAGATGATCGCCAAGAGCGAGCAGGAAATCGGCAAGGCTACCGCCAAG
TACTTCTTCTACAGCAACATCATGAACTTTTTCAAGACCGAGATTACCCTGGCCAACGGCG
AGATCCGGAAGCGGCCTCTGATCGAGACAAACGGCGAAACAGGCGAGATCGTGTGGGATA
AGGGCCGGGACTTTGCCACCGTGCGGAAAGTGCTGTCTATGCCCCAAGTGAATATCGTGA
AAAAGACCGAGGTGCAGACAGGCGGCTTCAGCAAAGAGTCTATCCTGCCCAAGAGGAACA
GCGACAAGCTGATCGCCAGAAAGAAGGACTGGGACCCTAAGAAGTACGGCGGCTTCGACA
GCCCCACCGTGGCCTATTCTGTGCTGGTGGTGGCCAAAGTGGAAAAGGGCAAGTCCAAGA
AACTGAAGAGTGTGAAAGAGCTGCTGGGGATCACCATCATGGAAAGAAGCAGCTTCGAGA
AGAATCCCATCGACTTTCTGGAAGCCAAGGGCTACAAAGAAGTGAAAAAGGACCTGATCAT
CAAGCTGCCTAAGTACTCCCTGTTCGAGCTGGAAAACGGCCGGAAGAGAATGCTGGCCTC
TGCCGGCGAACTGCAGAAGGGAAACGAACTGGCCCTGCCCTCCAAATATGTGAACTTCCT
GTACCTGGCCAGCCACTATGAGAAGCTGAAGGGCTCCCCCGAGGATAATGAGCAGAAACA
GCTGTTTGTGGAACAGCACAAACACTACCTGGACGAGATCATCGAGCAGATCAGCGAGTTC
TCCAAGAGAGTGATCCTGGCCGACGCTAATCTGGACAAGGTGCTGAGCGCCTACAACAAG
CACAGAGACAAGCCTATCAGAGAGCAGGCCGAGAATATCATCCACCTGTTTACCCTGACCA
ATCTGGGAGCCCCTGCCGCCTTCAAGTACTTTGACACCACCATCGACCGGAAGAGGTACAC
CAGCACCAAAGAGGTGCTGGACGCCACCCTGATCCACCAGAGCATCACCGGCCTGTACGA
GACACGGATCGACCTGTCTCAGCTGGGAGGCGACGCCTATCCCTATGACGTGCCCGATTATG
CCAGCCTGGGCAGCGGCTCCCCCAAGAAAAAACGCAAGGTGGAAGATCCTAAGAAAAAGCGGA
AAGTGGACGGCGGAGGGCCGAGCTCTGGCGCACCCCCACCAAGTGGAGGGTCTCCTGCCGGGTC
CCCAACATCTACTGAAGAAGGCACCAGCGAATCCGCAACGCCCGAGTCAGGCCCTGGTACCTCC
ACAGAACCATCTGAAGGTAGTGCGCCTGGTTCCCCAGCTGGAAGCCCTACTTCCACCGAAGAAG
GCACGTCAACCGAACCAAGTGAAGGATCTGCCCCTGGGACCAGCACTGAACCATCTGAGATGAA
CAATTCCCAGGGAAGAGTGACCTTCGAGGACGTGACCGTGAACTTCACCCAAGGCGAGTGGCAGA
GACTGAACCCCGAGCAGAGAAACCTGTACCGGGACGTGATGCTGGAAAACTACAGCAACCTGGTG
TCCGTCGGCCAGGGCGAGACAACAAAGCCTGACGTGATCCTGCGGCTGGAACAGGGCAAAGAGCC
TTGGCTGGAAGAGGAAGAGGTGCTCGGATCTGGCAGAGCCGAGAAGAATGGCGATATCGGCGGA
CAGATCTGGAAGCCAAAGGATGTGAAAGAGAGTCTCAGCGGCAGCGAGACCCCCGGTACCAGCG
AGGGCGGCCCTAGCAGCGGCGCCCCTCCCCCTAGCGGCGGCAGCCCTGCCGGATCCCCTACAAG
CACCGAAGAAGGCACAAGCGAGTCCGCTACCCCTGAGTCCGGCCCTGGCACCTCCACAGAGCCT
AGCGAGGGATCCGCTCCTGGATCCCCTGCCGGAAGCCCTACCTCCACCGAGGAGGGCACAAGCA
CCGAGCCTTCCGAGGGCAGCGCCCCCGGAACAAGCACCGAACCTAGCGAGAGCGCCACCCCCG
AGAGCATGTTCGAAACCGTGCCTGTGTGGAGGAGACAGCCAGTCCGGGTGCTGTCCCTTTTTGAAGAC
ATCAAGAAAGAGCTGACGAGTTTGGGCTTTTTGGAAAGTGGTTCTGACCCGGGACAACTGAAGCATGT
GGTTGATGTCACAGACACAGTGAGGAAGGATGTGGAGGAGTGGGGACCCTTCGATCTTGTGTACGGC
GCCACACCTCCCCTGGGCCACACCTGTGACCGTCCTCCCAGCTGGTACCTGTTCCAGTTCCACCGGCT
CCTGCAGTACGCACGGCCCAAGCCAGGCAGCCCCAGGCCCTTCTTCTGGATGTTCGTGGACAATCTG
GTGCTGAACAAGGAAGACCTGGACGTCGCATCTCGCTTCCTGGAGATGGAGCCAGTCACCATCCCAGA
TGTCCACGGCGGATCCTTGCAGAATGCTGTCCGCGTGTGGAGCAACATCCCAGCCATAAGGAGCAGCA
GGCACTGGGCTCTGGTTTCGGAAGAAGAATTGTCCCTGCTGGCCCAGAACAAGCAGAGCTCGAAGCTC
GCGGCCAAGTGGCCCACCAAGCTGGTGAAGAACTGCTTTCTCCCCCTAAGAGAATATTTCAAGTATTTT
TCAACAGAACTCACTTCCTCTTTATCTAGCGGCAATAGTAACGCTAACAGCCGCGGGCCGAGCTT
CAGCAGCGGCCTGGTGCCGTTAAGCTTGCGCGGCAGCCATATGAACCATGACCAGGAATTTGACCC
CCCAAAGGTTTACCCACCTGTGCCAGCTGAGAAGAGGAAGCCCATCCGCGTGCTGTCTCTCTTTGATG
GGATTGCTACAGGGCTCCTGGTGCTGAAGGACCTGGGCATCCAAGTGGACCGCTACATTGCCTCCGA
GGTGTGTGAGGACTCCATCACGGTGGGCATGGTGCGGCACCAGGGAAAGATCATGTACGTCGGGGAC
GTCCGCAGCGTCACACAGAAGCATATCCAGGAGTGGGGCCCATTCGACCTGGTGATTGGAGGCAGTC

CCTGCAATGACCTCTCCATTGTCAACCCTGCCCGCAAGGGACTTTATGAGGGTACTGGCCGCCTCTTCT
TTGAGTTCTACCGCCTCCTGCATGATGCGCGGCCCAAGGAGGGAGATGATCGCCCCTTCTTCTGGCTC
TTTGAGAATGTGGTGGCCATGGGCGTTAGTGACAAGAGGGACATCTCGCGATTTCTTGAGTCTAACCCC
GTGATGATTGACGCCAAAGAAGTGTCTGCTGCACACAGGGCCCGTTACTTCTGGGGTAACCTTCCTGG
CATGAACAGGCCTTTGGCATCCACTGTGAATGATAAGCTGGAGCTGCAAGAGTGTCTGGAGCACGGCA
GAATAGCCAAGTTCAGCAAAGTGAGGACCATTACCACCAGGTCAAACTCTATAAAGCAGGGCAAAGACC
AGCATTTCCCCGTCTTCATGAACGAGAAGGAGGACATCCTGTGGTGCACTGAAATGGAAAGGGTGTTT
GGCTTCCCCGTCCACTACACAGACGTCTCCAACATGAGCCGCTTGGCGAGGCAGAGACTGCTGGGCC
GATCGTGGAGCGTGCCGGTCATCCGCCACCTCTTCGCTCCGCTGAAGGAATATTTTGCTTGTGTGCTC
GAGGCTACTAACTTCAGCCTGCTGAAGCAGGCTGGAGACGTGGAGGAGAACCCTGGACCTATGC
ATGTGAGCGAGCTGATTAAGGAGAACATGCACATGAAGCTGTACATGGAGGGCACCGTGGACAAC
CATCACTTCAAGTGCACATCCGAGGGCGAAGGCAAGCCCTACGAGGGCACCCAGACCATGAGAAT
CAAGGTGGTCGAGGGCGGCCCTCTCCCCTTCGCCTTCGACATCCTGGCTACTAGCTTCCTCTACGG
CAGCAAGACCTTCATCAACCACACCCAGGGCATCCCCGACTTCTTCAAGCAGTCCTTCCCTGAGGG
CTTCACATGGGAGAGAGTCACCACATACGAAGACGGGGGCGTGCTGACCGCTACCCAGGACACCA
GCCTCCAGGACGGCTGCCTCATCTACAACGTCAAGATCAGAGGGGTGAACTTCACATCCAACGGC
CCTGTGATGCAGAAGAAAACACTCGGCTGGGAGGCCTTCACCGAAACGCTGTACCCCGCTGACGG
CGGCCTGGAAGGCAGAAACGACATGGCCCTGAAGCTCGTGGGCGGGAGCCATCTGATCGCAAACA
TCAAGACCACATATAGATCCAAGAAACCCGCTAAGAACCTCAAGATGCCTGGCGTCTACTATGTGG
ACTACAGACTGGAAAGAATCAAGGAGGCCAACAACGAGACCTACGTCGAGCAGCACGAGGTGGCA
GTGGCCAGATACTGCGACCTCCCTAGCAAACTGGGGCACAAGCTTAATTAA(SEQ ID NO:107).

The amino acid sequence of the editing tool EpiRegC (dCas9-ZIM3 KRAB-DNMT3L-Dnmt3a) (italic/underlined italic: Dnmt3a/Dnmt3l, bold: dCas9, bold italic: KRAB, underlined bold italic: blue fluorescent protein, standard font: linker sequence, *: any amino acid) is as follows:

MPKKKRKVMDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHERHPIFG
NIVDEVAYHEKYPTIYHLRKKLVDSTDKADLRLIYLALAHMIKFRGHFLIEGDLNPDNSDVDKL
FIQLVQTYNQLFEENPINASGVDAKAILSARLSKSRRLENLIAQLPGEKKNGLFGNLIALSLGLTP
NFKSNFDLAEDAKLQLSKDTYDDDLDNLLAQIGDQYADLFLAAKNLSDAILLSDILRVNTEITKA
PLSASMIKRYDEHHQDLTLLKALVRQQLPEKYKEIFFDQSKNGYAGYIDGGASQEEFYKFIKPI
LEKMDGTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDKNLPNEKV
LPKHSLLYEYFTVYNELTKVKYVTEGMRKPAFLSGEQKKAIVDLLFKTNRKVTVKQLKEDYF
KKIECFDSVEISGVEDRFNASLGTYHDLLKIIKDKDFLDNEENEDILEDIVLTLTLFEDREMIEER
LKTYAHLFDDKVMKQLKRRRYTGWGRLSRKLINGIRDKQSGKTILDFLKSDGFANRNFMQLI
HDDSLTFKEDIQKAQVSGQGDSLHEHIANLAGSPAIKKGILQTVKVVDELVKVMGRHKPENIVI
EMARENQTTQKGQKNSRERMKRIEEGIKELGSQILKEHPVENTQLQNEKLYLYYLQNGRDMY
VDQELDINRLSDYDVDAIVPQSFLKDDSIDNKVLTRSDKNRGKSDNVPSEEVVKKMKNYWRQL
LNAKLITQRKFDNLTKAERGGLSELDKAGFIKRQLVETRQITKHVAQILDSRMNTKYDENDKLI
REVKVITLKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEIVWDKG
RDFATVRKVLSMPQVNIVKKTEVQTGGFSKESILPKRNSDKLIARKKDWDPKKYGGFDSPTVA
YSVLVVAKVEKGKSKKLKSVKELLGITIMERSSFEKNPIDFLEAKGYKEVKKDLIIKLPKYSLFE
LENGRKRMLASAGELQKGNELALPSKYVNFLYLASHYEKLKGSPEDNEQKQLFVEQHKHYLD
EIIEQISEFSKRVILADANLDKVLSAYNKHRDKPIREQAENIIHLFTLTNLGAPAAFKYFDTTIDR
KRYTSTKEVLDATLIHQSITGLYETRIDLSQLGGDAYPYDVPDYASLGSGSPKKKRKVEDPKKKR
KVDGGGPSSGAPPPSGGSPAGSPTSTEEGTSESATPESGPGTSTEPSEGSAPGSPAGSPTSTEEGTSTEPS
EGSAPGTSTEPSEMNNSQGRVTFEDVTVNFTQGEWQRLNPEQRNLYRDVMLENYSNLVSVGQGETT
KPDVILRLEQGKEPWLEEEEVLGSGRAEKNGDIGGQIWKPKDVKESLSGSETPGTSEGGPSSGAPPPS
GGSPAGSPTSTEEGTSESATPESGPGTSTEPSEGSAPGSPAGSPTSTEEGTSTEPSEGSAPGTSTEPSESA
TPESMFETVPVWRRQPVRVLSLFEDIKKELTSLGFLESGSDPGQLKHVVDVTDTVRKDVEEWGPFDLVYG
ATPPLGHTCDRPPSWYLFQFHRLLQYARPKPGSPRPFFWMFVDNLVLNKEDLDVASRFLEMEPVTIPDVH
GGSLQNAVRVWSNIPAIRSSRHWALVSEEELSLLAQNKQSSKLAAKWPTKLVKNCFLPLREYFKYFSTELTSS
LSSGNSNANSRGPSFSSGLVPLSLRGSHMNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSPCNDLSIVNPARKGLYE
GTGRLFFEFYRLLHDARPKEGDDRPFFWLFENVVAMGVSDKRDISRFLESNPVMIDAKEVSAAHRARYFW
GNLPGMNRPLASTVNDKLELQECLEHGRIAKFSKVRTITTRSNSIKQGKDQHFPVFMNEKEDILWCTEMER
VFGFPVHYTDVSNMSRLARQRLLGRSWSVPVIRHLFAPLKEYFACVLEATNFSLLKQAGDVEENPGPMHV
SELIKENMHMKLYMEGTVDNHHFKCTSEGEGKPYEGTQTMRIKVVEGGPLPFAFDILATSFLYGSK
TFINHTQGIPDFFKQSFPEGFTWERVTTYEDGGVLTATQDTSLQDGCLIYNVKIRGVNFTSNGPVMQ

KKTLGWEAFTETLYPADGGLEGRNDMALKLVGGSHLIANIKTTYRSKKPAKNLKMPGVYYVDYRLER
IKEANNETYVEQHEVAVARYCDLPSKLGHKLN*(SEQ ID NO:108).

(3) Detection Results: The gRNA used in the positive control (PC) group in this example directly targets EGFP (SEQ ID NO: 112). When the expression of the target LPA gene is suppressed, the intensity of EGFP will correspondingly decrease. The proportion of cells with low green fluorescence among the cell population co-transfected with blue and red fluorescence was detected by flow cytometry (Figure 1B). Then, the proportion of samples transfected with different gRNAs was subtracted by the proportion of the NT gRNA (SEQ ID NO: 113) samples, which do not target the regulatory region, to obtain the relative regulation efficiency of different editing tools using different gRNAs. From the regulation efficiency results shown in Figure 1C, it can be seen that gRNAs shown in SEQ ID NOs: 2, 3, 4, 7, 10, 11, and 12 (the 5'-end of their targeting regions start at 222 bp upstream, 330 bp downstream, 344 bp downstream, 408 bp upstream, 163 bp upstream, 207 bp upstream, and 266 bp downstream of TSS, respectively) combined with the epigenetic editing tools of this example can effectively suppress the expression level of the LPA gene.

**Table 1. sgRNAs targeting the human LPA gene**

| SEQ ID NO: | Sequence | Position of the Target Sequence Relative to the Transcription Start Site (TSS) (+: Downstream of the TSS, -: Upstream of the TSS) | |
|---|---|---|---|
| | | 5'-end | 3'-end |
| 2 | AAGGAGACATAAAGGCAATG | -222 | -203 |
| 3 | AAAAGAGTGTGCCTACATGA | +330 | +349 |
| 4 | AGCCAGACTGGCCATCATGT | +344 | +363 |
| 7 | CCTGTTGGAAAGCTTGAGGG | -408 | -389 |
| 10 | GTGCAATGTCAATAGATGCT | -163 | -144 |
| 11 | CAATGTGGAGCAGCTGAGGG | -207 | -188 |
| 12 | GCACCGTGACAGTCTTCACG | +266 | +285 |

### Example 2

With reference to any nucleotide sequence shown in SEQ ID NOs: 1-50, a guide RNA in the epigenetic editing tool was constructed to target the target gene; meanwhile, based on the gene expression-regulating molecules provided in the present application, an epigenetic editing tool was constructed. For example, it may target the region within 500 bp upstream and downstream of the LPA transcription start site (TSS).

The guide RNA plasmid and the gene expression-regulating molecule plasmid were co-transfected into a mouse cell line. After 72 hours, the top 10% of GFP+ and mCherry+ cells were sorted by FACS. RT-QPCR experiments were conducted to assess the mRNA expression levels of the target gene.

The results show that the transfected cells showed a reduction in the expression of LPA expression products. For example, the transfected cells can show a reduction in the mRNA expression of LPA transcription. For example, the transfected cells can show a reduction in the expression of LPA protein products. For xample, using the editing method of the present application can alleviate diseases or conditions related to the LPA gene.

### Example 3

### Universality of Epigenetic Targets

The epigenetic editing tools of the present application were used for the expression regulation of targets. The domain in the gene expression-regulating molecule that has the function of binding to gene sequences can be replaced with known TALEN DNA binding domains, zinc finger domains, tetR DNA binding domains, meganucleases, and/or any CRISPR/Cas system nuclease domains in the art, such as dCas12 enzymes, etc.

The results show that the epigenetic targets of the present application are universal, and using various gene sequence binding domains has a high effect on regulating gene expression.

The detailed description provided above is for explanatory and illustrative purposes and is not intended to limit the scope of the appended claims. Various modifications to the embodiments described herein will be apparent to those skilled in the art and are reserved within the scope of the appended claims and their equivalents.

## Claims

1. A method for regulating the expression and/or activity of the LPA gene, comprising providing a gene expression-regulating molecule or a nucleic acid encoding the gene expression-regulating molecule, wherein the gene expression-regulating molecule has the function of regulating the expression of the LPA gene without altering its gene sequence.

2. A method for treating and/or alleviating a condition related to abnormal expression and/or activity of the LPA gene, comprising providing a gene expression-regulating molecule or a nucleic acid encoding the gene expression-regulating molecule, wherein the gene expression-regulating molecule has the function of regulating the expression of the LPA gene without altering its gene sequence.

3. The method of any one of claims 1-2, wherein the gene expression-regulating molecule comprises a first functional domain providing modification of at least one nucleotide in the vicinity of the LPA gene and/or within the LPA gene regulatory elements.

4. The method of claim 3, wherein the modification of at least one nucleotide comprises methylation modification.

5. The method of any one of claims 3-4, wherein the regulatory elements comprise a core promoter, a proximal promoter, a distal enhancer, a silencer, an insulator element, a boundary element, and/or a locus control region.

6. The method of any one of claims 3-5, wherein the first functional domain comprises one or more of DNA methyltransferase, DNA demethylase, and a functional active fragment thereof.

7. The method of claim 6, wherein the DNA methyltransferase comprises one or more of DNMT 3A, DNMT 3B, DNMT 3L, DNMT 1, and DNMT 2.

8. The method of claim 7, wherein the DNMT 3A is derived from mouse.

9. The method of any one of claims 7-8, wherein the DNMT 3L is derived from human and/or mouse.

10. The method of any one of claims 7-9, wherein the DNMT 3A and the DNMT 3L are directly and/or indirectly linked.

11. The method of any one of claims 1-10, wherein the gene expression-regulating molecule comprises a second functional domain comprising a zinc finger protein-based transcription factor or a functional active fragment thereof, or a substance capable of modifying histone.

12. The method of claim 11, wherein the second functional domain comprises Krab.

13. The method of any one of claims 11-12, wherein the second functional domain comprises ZIM3 Krab or KOX1 Krab.

14. The method of claim 11, wherein the second functional domain comprises one or more of histone methyltransferase, histone demethylase, histone acetyltransferase, histone deacetylase, and a functional active fragment thereof.

15. The method of any one of claims 11-14, wherein the gene expression-regulating molecule comprises the first functional domain and the second functional domain, with the first functional domain is directly or indirectly linked to one end of the second functional domain, or the first functional domain is directly and/or indirectly linked to both ends of the second functional domain.

16. The method of any one of claims 1-15, wherein the gene expression-regulating molecule comprises a DNA binding domain.

17. The method of any one of claims 1-16, wherein the gene expression-regulating molecule comprises one or more DNA binding domains selected from TALEN domain, zinc finger domain, and protein domain of CRISPR/Cas system.

18. The method of any one of claims 1-17, wherein the gene expression-regulating molecule comprises Cas enzyme.

19. The method of any one of claims 1-18, wherein the gene expression-regulating molecule comprises a Cas enzyme that substantially incapable of nuclease activity.

20. The method of any one of claims 1-19, wherein the gene expression-regulating molecule comprises dCas9 enzyme.

21. The method of any one of claims 16-20, wherein the first functional domain and the second functional domain are directly or indirectly linked to one end of the DNA binding domain, or the first functional domain and the second functional domain are directly and/or indirectly linked to both ends of the DNA binding domain.

22. The method of any one of claims 1-21, wherein the gene expression-regulating molecule is capable of binding to a DNA region within 500 bp upstream and/or downstream of the transcription start site (TSS) of the LPA gene, or a fragment thereof.

23. The method of any one of claims 1-22, wherein the gene expression-regulating molecule is capable of binding to a DNA region where SEQ ID NO: 51 is located, or a fragment thereof.

24. The method of any one of claims 1-23, wherein the gene expression-regulating molecule is capable of binding to one or more DNA regions in the vicinity of the transcription start site (TSS) of the LPA gene as described below: between 410 bp upstream and 380 bp upstream of the TSS, between 230 bp upstream and 140 bp upstream of the TSS, and between 260 bp downstream and 370 bp downstream of the TSS.

25. The method of any one of claims 1-24, wherein the gene expression-regulating molecule is capable of binding to one or more DNA regions in the vicinity of the transcription start site (TSS) of the LPA gene as described below: between 410 bp upstream and 380 bp upstream of the TSS, between 230 bp upstream and 190 bp upstream of the TSS, between 170 bp upstream and 140 bp upstream of the TSS, between 260 bp downstream and 290 bp downstream of the TSS, and between 330 bp downstream and 370 bp downstream of the TSS.

26. The method of any one of claims 1-25, wherein the method comprises providing a nucleic acid-binding molecule comprising a sequence of any one of SEQ ID NOs: 1-50.

27. The method of claim 26, wherein the gene expression-regulating molecule and/or the nucleic acid-binding molecule are formulated in a same delivery carrier or in different delivery carriers.

28. The method of claim 27, wherein the delivery carrier comprises liposome and/or lipid nanoparticle.

29. The method of any one of claims 26-28, wherein the expression-regulating molecule and/or the nucleic acid-binding molecule are formulated in a same recombinant vector or in different recombinant vectors.

30. The method of claim 29, wherein the recombinant vector comprises viral vector.

31. The method of any one of claims 29-30, wherein the recombinant vector comprises adeno-associated viral vector.

32. The method of any one of claims 1-31, wherein the gene expression-regulating molecule comprises a nuclear localization sequence.

33. The method of claim 32, wherein the nuclear localization sequence comprises amino acid with positively charged group.

34. The method of any one of claims 32-33, wherein the nuclear localization sequence is located at the N-terminal and/or C-terminal of the first functional domain, the N-terminal and/or C-terminal of the second functional domain, and/or the N-terminal and/or C-terminal of the DNA binding domain.

35. A nucleic acid-binding molecule, comprising a sequence of any one of SEQ ID NOs: 1-50.

36. A gene expression-regulating molecule, having the function of regulating the expression of the LPA gene without altering its gene sequence.

37. The gene expression-regulating molecule of claim 36, wherein the gene expression-regulating molecule is the gene expression-regulating molecule provided in the method of any one of claims 1-34.

38. The gene expression-regulating molecule of any one of claims 36-37, wherein the gene expression-regulating molecule and/or the nucleic acid-binding molecule are formulated in a same delivery carrier or in different delivery carriers, the nucleic acid-binding molecule comprises a sequence of any one of SEQ ID NOs: 1-50.

39. The gene expression-regulating molecule of claim 38, wherein the delivery carrier comprises liposome and/or lipid nanoparticle.

40. The gene expression-regulating molecule of any one of claims 36-39, wherein the expression-regulating molecule and/or the nucleic acid-binding molecule are formulated in a same recombinant vector or in different recombinant vectors, the nucleic acid-binding molecule comprises a sequence of any one of SEQ ID NOs: 1-50.

41. The gene expression-regulating molecule of claim 40, wherein the recombinant vector comprises viral vector.

42. The gene expression-regulating molecule of any one of claims 40-41, wherein the recombinant vector comprises adeno-associated viral vector.

43. A nucleic acid encoding the nucleic acid-binding molecule of claim 35 and/or encoding the gene expression-regulating molecule of any one of claims 36-42.

44. A recombinant vector comprising the nucleic acid of claim 43.

45. A delivery carrier comprising the nucleic acid-binding molecule of claim 35, the gene expression-regulating molecule of any one of claims 36-42, the nucleic acid of claim 43, and/or the recombinant vector of claim 44, and optionally comprising liposomes and/or lipid nanoparticles.

46. A composition comprising the nucleic acid-binding molecule of claim 35, the gene expression-regulating molecule of any one of claims 36-42, the nucleic acid of claim 43, the recombinant vector of claim 44, and/or the delivery carrier of claim 45.

47. A cell comprising the nucleic acid-binding molecule of claim 35, the gene expression-regulating molecule of any one of claims 36-42, the nucleic acid of claim 43, the recombinant vector of claim 44, the delivery carrier of claim 45, and/or the composition of claim 46.

48. A kit comprising the nucleic acid-binding molecule of claim 35, the gene expression-regulating molecule of any one of claims 36-42, the nucleic acid of claim 43, the recombinant vector of claim 44, the delivery carrier of claim 45, the composition of claim 46, and/or the cell of claim 47.
